# EUROPEAN PATENT SPECIFICATION

(11) **EP 1 212 440 B1**
(45) Date of publication and mention of the grant of the patent: **02.04.2008**
(21) Application number: 00958901.1
(22) Date of filing: 13.09.2000
(51) Int. Cl.: C12N 15/82, C08B 30/00, C08B 30/12, C12N 9/10, A01H 5/00

(54) **PLANTS HAVING REDUCED ACTIVITY IN TWO OR MORE STARCH-MODIFYING ENZYMES**
PFLANZEN MIT VERRINGERTER AKTIVITÄT IN ZWEI ODER MEHR STÄRKE-MODIFIZIERENDEN ENZYMEN
PLANTES DONT UNE OU PLUSIEURS ENZYMES DE MODIFICATION DE L'AMIDON PRESENTENT UNE ACTIVITE REDUITE

(30) Priority: 15.09.1999 GB 9921830
(43) Date of publication of application: 12.06.2002
(73) Proprietor: NATIONAL STARCH AND CHEMICAL INVESTMENT HOLDING CORPORATION, Wilmington, Delaware 19850 (US)
(72) Inventor: JOBLING, Stephen Alan, Huntingdon, Cambridgeshire PE19 3QQ (GB); SCHWALL, Gerhard, Peter, D-57392 Schmallenberg (DE); WESTCOTT, Roger, John, Wellingborough, Northampton NN8 1LW (GB)
(74) Representative: Lipscombe, Martin John
(86) International application number: PCT/GB2000/003522
(87) International publication number: WO 2001/019975

(56) References cited:
- EP-A- 0 788 735
- WO-A-00/08184
- WO-A-00/58366
- WO-A-94/09144
- WO-A-96/15248
- WO-A-97/20040
- WO-A-98/44780
- US-A- 4 428 972
- US-A- 5 954 883
- AHAMED N T ET AL: "Physicochemical and functional properties of Chenopodium quinoa starch" CARBOHYDRATE POLYMERS,APPLIED SCIENCE PUBLISHERS, LTD. BARKING,GB, vol. 31, no. 1, 1 September 1996 (1996-09-01), pages 99-103, XP004063522 ISSN: 0144-8617
- YASUI T ET AL: "AMYLOSE AND LIPID CONTENTS, AMYLOPECTIN STRUCTURE, AND GELATINISATION PROPERTIES OF WAXY WHEAT (TRITICUM AESTIVUM) STARCH" JOURNAL OF CEREAL SCIENCE,XX,ACADEMIC PRESS LTD, vol. 24, no. 2, 1996, pages 131-137, XP000863063 ISSN: 0733-5210
- EDWARDS ET AL: "A combined reduction in activity of starch synthases II and III of potato has novel effects on the starch of tubers" PLANT JOURNAL,BLACKWELL SCIENTIFIC PUBLICATIONS, OXFORD,GB, vol. 17, no. 3, February 1999 (1999-02), pages 251-261, XP002115084 ISSN: 0960-7412 cited in the application
- EDWARDS A ET AL: "BIOCHEMICAL AND MOLECULAR CHARACTERIZATION OF A NOVEL STARCH SYNTHASE FROM POTATO TUBERS" PLANT JOURNAL,GB,BLACKWELL SCIENTIFIC PUBLICATIONS, OXFORD, vol. 8, no. 2, 1995, pages 283-294, XP000566770 ISSN: 0960-7412 cited in the application
- VISSER R G F ET AL: "INHIBITION OF THE EXPRESSION OF THE GENE FOR GRANULE-BOUND STARCH SYNTHASE IN POTATO BY ANTISENSE CONSTRUCTS" MOLECULAR AND GENERAL GENETICS,DE,SPRINGER VERLAG, BERLIN, vol. 225, 1 February 1991 (1991-02-01), pages 289-296, XP000611638 ISSN: 0026-8925

## Description

### Field of the Invention

The present invention relates to plant modification, especially modification by manipulating the activity of a combination of plant enzymes having starch synthase activity to alter the nature of starch obtainable from the plant. In particular, it relates to manipulation of activity of the plant enzymes granule bound starch synthase I (GBSSI) and one, or both, of starch synthase II (SSII) and starch synthase III (SSIII); and modified plants obtained thereby.

### Background of the Invention

Starch is the primary form of carbon reserve in plants, constituting 50% or more of the dry weight in many storage organs, e.g. tubers, seeds. Starch consists of two major components: amylose, an essentially linear polymer of (1→4) α-D-glucopyranose units; and amylopectin, a branched polymer of shorter chains of (1→4) α-D-glucopyranose units with (1→6) α branches.

Starch is of importance in a variety of applications, such as in the paper, textiles and adhesives industries. Commonly, native starches obtained from storage organs of plants such as cereal endosperms, potato tubers and pea embryos are further modified, generally by either chemical or physical means, to produce starches having improved properties better suited to the particular intended application. Commercial interest has been directed, for example, to methods for modifying or manipulating the freeze-thaw stability of starches. The use of native (i.e. non-*in vitro* modified) starches in products which undergo freezing and thawing is severely limited due to the undesirable textural changes which occur in most starch-thickened systems following such freeze-thaw treatment. These changes have been ascribed to a phase separation as a result of retrogradation (Albrecht et al 1960 Food Technology 14, 57-63).

By way of explanation, when native starch is heated in an aqueous environment, the starch granules tend to swell. During this swelling, amylose tends to solubilize and leak out into the surrounding water. Upon cooling the amylose chains re-associate to form a gelatinous paste. This reassociation is known as retrogradation. Amylopectin is generally less susceptible to retrogradation.

Retrogradation can be prevented by chemical modification (e.g. hydroxypropylation) which interferes with the association between molecules or portions of the same molecule (see, for example, EP-A-0796868). In combination with crosslinking, these modifications can improve the freeze-thaw stability of starches (Kim & Eliasson J. Textural Studies (1993) 24, 199-213). The "freeze-thaw stability" of a starch relates to the resistance of the starch (when formed into a paste) to changes (particularly decreases) in viscosity following exposure to one or more cycles of freeze/thawing. Retrogradation of starches is closely related to the formation of inter-chain double helices and occurs over different time scales for the amylose and amylopectin components, amylose retrogradation being more rapid than amylopectin. Thus, where short term stability of starch pastes is required, "waxy" starches (i.e. starches which are amylose-free, or substantially so) are often utilised but even these show retrogradation upon freezing and thawing. However, in maize, certain waxy double mutant plants (i.e. plants with the waxy phenotype but also containing one or more additional genetic mutations) give rise to starch showing improved freeze-thaw stability e.g. waxy-sugary2 and waxy-shrunken1 phenotypes (US 4,428,972 and US 4,767,849).

It would be desirable commercially to provide plants which intrinsically produce native starches having the desired properties, thereby obviating the need for additional, costly and generally inefficient, *in vitro* modification steps. To this end, considerable interest has been expressed in the art in studying the starch biosynthetic pathway in plants, with the aim of modifying the plant genome to produce starches with novel and advantageous properties.

Approaches to modifying the starch biosynthetic pathway in plants using recombinant DNA technology have recently been described in the literature. In particular, methods based on manipulating the activity of plant enzymes having either starch branching (SBE) or starch synthase (SS) activity, generally regarded as the most important starch-synthesising enzymes, have been studied.

WO 06/34968 discloses a nucleotide sequence encoding an effective portion of a Class A starch branching enzyme (SBE) obtainable from potato plants, which sequence can be introduced, conveniently linked in an antisense orientation to a suitable promoter and preferably together with an effective portion of a sequence encoding a Class B starch branching enzyme, into a plant to alter the characteristics of the plant. (By way of explanation, starch-branching enzymes can be identified as Class A or Class B, depending on their amino acid sequence; for a review, see Burton et al, 1995 The Plant Journal 7, 3-15). It is disclosed that starch extracted from a plant so transformed has an increased amylose content compared to starch extracted from a similar but unaltered plant.

Isoforms of starch synthase are found in the storage organs of most species of plants and there is currently much interest in characterising them and studying their role in starch synthesis. Starch synthases are classified into two groups depending on whether they are found associated with the starch granule (so called granule bound starch synthases, GBSS) or whether they are predominantly found in the stroma of the plastid soluble starch synthases, SS). GBSSI is known to be required for the formation of amylose since mutants lacking this activity produce starch having substantially no amylose (so called "waxy starch"). Examples of GBSSI mutants include the low amylose locus (*lam*) of pea and the *waxy* locus in maize, (Denyer et al, Plant Cell Environ. 18, 1019-1026, 1995; Shure et al, Cell 35, 225-233, 1983). Plants where expression of this gene has been eliminated by antisense technology also have reduced amylose contents (e.g. potato, Visser et al Mol. Gen. Genet. 225, 289-296, 1991). EP 0788735 relates to the genetic modification of potato plants, and describes the theoretical inhibition of GBSSI expression in such plants.

Multiple isoforms of soluble starch synthases have been described. For example, in pea the rug5 locus was recently shown to be a mutation in the major soluble isoform of starch synthase (SSII) (Craig et al, Plant Cell 10 413-426, 1998) and caused an increase in short chains and a decrease in intermediate chains of amylopectin. The recent cloning of the *dull1* gene in maize identified that locus as a starch synthase, most likely SSII (Gao et al, Plant Cell 10, 399-412, 1998) and mutants at this locus have changes in both amylose content and degree of branching of amylopectin (Wang et al, Cereal Chem. 70, 171-179, & 521-525 1993). In addition, the cloning of starch synthases from maize (Harn et al. Plant Mol. Biol. 37, 629-637 1998; Knight et al, Plant J. 14, 613-622 1998; WO97/44472; and WO97/20936) and wheat (WO97/45545) have been described. The role of each isoform in the control of starch synthesis and structure is unclear at present since the contribution of each isoform to the total activity varies considerably between species.

In potato, starch synthase III (SSIII), a largely soluble isoform having a molecular mass as judged by SDS-PAGE in the range of 100-140 kDa, (Marshall et al, Plant Cell 8, 1121-1135, 1996) is one of three soluble isoforms of starch synthase, each encoded by a different gene. Starch synthase II (SSII), formerly known as GBSSII (Edwards et al, Plant J. 8, 283-294, 1995) is found in both soluble and granule bound forms and has an apparent molecular weight of approximately 78kDa. Potato plants either lacking these other isoforms or having reduced isoform activity have been generated and the effects on the properties of the starch obtained therefrom have been studied.

Reductions in the amount of both soluble and granule-bound SSII protein via expression of antisense RNA were found to have little or no effect on the total (soluble and granule-bound) starch synthase activity of the tuber, the amount of starch, or the amylose to amylopectin ratio of the starch (see Edwards et al, 1995 cited above; and Koßmann et al Macromol. Symp. 120, 29-38, 1997).

Marked effects on the properties of starch, in particular a reduction in the viscosity onset temperature compared to untransformed material has been observed when potato plants are modified by manipulation of starch synthase III (SSIII) (see EP-A-0779363; and Marshall *et al* 1996, cited above). A reduction in the onset temperature for gelatinisation of starch extracted from transformed potato plants of 5°C, compared to starch extracted from equivalent, non-transformed plants, was reported. In addition to the differences in starch properties, altered starch granule morphology and reductions in soluble starch synthase activity in the order of 80% were reported. WO 96/15248 discloses potato plants transformed with a portion of either one of two cDNA clones, denoted SSSA and SSSB, which are said to encode isoforms of potato soluble starch synthases.

Recently, it was found that a combined reduction of SSII and SSIII in potato tubers had a much greater effect on starch structure and properties than could have been predicted from reductions in the individual isoforms (WO 99/66050; Edwards et al, Plant J. 17, 251-261, 1999). A reduction in the onset temperature for gelatinisation of starch extracted from transformed potato plants of at least 10°C. compared to starch extracted from equivalent, non-transformed plants was reported. In addition, the proportion of amylose in the starch (as assayed by gel permeation chromatography) increased significantly and the chain length distribution of amylopectin was altered such that there were more short chains with a degree of polymerisation (DP) of 6-7 and less chains of DP15-20. These results were surprising and could not have been predicted from the prior art.

For example, Edwards et al (1999 Plant J. 17, 251-261) stated that:
"our results also indicate that the SSII and SSIII isoforms act in a synergistic manner, rather than independently, in the synthesis of amylopectin. The starch of all of the SII/SSIII lines is qualitatively unlike that of either the SSII or the SSIII lines with respect to granule morphology, amylopectin structure and gelatinisation behaviour. Its properties in these respects cannot be predicted from consideration of those of the starches of the SSII and SSIII lines".

Similarly, Lloyd et al., (1999 Biochem. J. 338, 515-521) found that: "the SSII and SSIII isoforms interact with each other in the production of amylopectin and, thus, when they are simultaneously reduced there is a synergistic effect on the amylopectin", and that "reduction in activity of both of these isoforms simultaneously ... leads to a gross alteration in the amylopectin".

There remains a continuing need for the development of improved methods for modulating or manipulating starch biosynthesis in plants with the aim of producing transformed plants and starches having improved properties. In particular, there is considerable commercial interest in the development of improved methods for producing transformed plants providing starches having improved freeze thaw stability since these are not currently available. Benefits resulting from the use of such plants in the preparation of starch products include improvements in quality and storage. However, the multiplicity of starch enzyme isoforms means it is difficult to assign particular roles or functions to particular isoforms. It is also unclear what, if any, interaction occurs between the various enzymes involved in starch synthesis. It is therefore impossible to predict from the prior art the effects of reducing the activity in a plant of a particular combination of such enzymes.

### Summary of the Invention

The present inventors have used recombinant DNA technology to produce plants in which the levels of expression of two or more different enzymes, each involved in starch synthesis in the plant, have been specifically inhibited.

Accordingly, in a first aspect, the invention provides a potato plant cell comprising introduced nucleic acid sequences, which sequences specifically inhibit the expression of granule bound starch synthase I (GBSSI) and at least one further starch synthase enzyme involved in starch synthesis in the potato plant cell. The invention also provides, in second and third aspects an equivalent potato plant, or the progeny thereof, respectively. References to a "plant" should be construed as including, where the context permits, reference to a part of a plant (especially a part of the plant rich in starch, such as a seed, tuber, etc).

Preferably, the said at least one further starch synthase enzyme which is specifically inhibited is starch synthase III (SSIII). In preferred embodiments the invention involves the specific inhibition of at least two further starch synthase enzymes, SSIII and SSII. Accordingly, in such embodiments the method involves the specific inhibition of expression of three or more different enzymes, each involved in starch synthesis in the cell. The invention is exemplified below by inhibition of SSIII and GBSSI, with or without inhibition of SSII, in potato plants.

In the examples below sequences corresponding to substantial parts of the potato SSIII and GBSSI genes, (and optionally the SSII gene) were introduced (into potato plants) operably linked in the antisense orientation to a promoter active in the plant, so as to cause transcription of the introduced sequences, resulting in specific inhibition of expression of the respective endogenous genes. Promoters suitable for this purpose in potato or other plants are well known to those skilled in the art and include, for example, the CaMV 355 promoter (especially when arranged in tandem), patatin promoter, GBSSI promoter, Nos promoter and the like. The choice of promoter may be determined, at least in part, by the plant and/or tissue in which the enzyme expression is to be inhibited.

As an alternative to such methods, which may be generally referred to as "antisense inhibition", it should prove possible to reduce levels of expression of enzymes by "sense suppression" in which nucleic acid sequences, corresponding to incomplete portions of the genes to be inhibited, are introduced into the plant operably linked in the sense orientation to a promoter active in the plant. This phenomenon is well-known to those skilled in the art and is reviewed for example, by Grant (1999 Cell 96, 303-306); and Jorgenson (1999 Trends in Genetics 15, 11-12).

In preferred embodiments, the enzymes whose expression is to be inhibited comprise SSII, SSIII and GBSSI. However, inhibition of other enzymes, in addition to GBSSI and a further starch synthase enzyme, may also be considered. Other such enzymes which it may be desired to inhibit will normally, but not necessarily, be involved in starch synthesis. Examples include isoamylases, pullulanases, and starch branching enzymes (see, e.g. W 99/12950; WO 96/34968).

Whilst it may be possible to inhibit expression of, for example, SSII in maize plants using a nucleic acid sequence derived from the potato SSII gene it will generally be desirable, for optimum results, to use sequences derived from the corresponding endogenous gene whose expression it is desired to inhibit. The homologues of the potato SSII, SSIII and GBSS I genes are known for several plants. For example, homologues of the potato SSII gene have been desribed for pea (Dry et al, 1992 Plant J. 2, 193-202); cassava (Munyikwa et al, 1997 Euphytica 96, 65-75) and maize (Harn et al, 1998 Plant Mol. Biol. 37, 639-649). Homologues of the potato SSIII gene have been described for maize (Gao et al, 1998 Plant Cell 10, 399-412) and pea (Craig et al, 1998 Plant Cell 10, 413-426; Tomlinson et al, 1998 Planta 204, 86-92). Those skilled in the art will appreciate that the sequence used to inhibit the endogenous gene need not necessarily be a naturally occurring sequence but could be, for example, a synthetic sequence (e.g. a consensus sequence based on analysis of the sequences of starch synthase genes from different plants).

It will be apparent to the person skilled in the art that he introduced sequence, whether in the sense or in the antisense orientation, need not be identical with the endogenous gene whose expression is to be inhibited in order to obtain the benefits of the invention. Thus, the invention encompasses the use of sequences which are functionally equivalent to the endogenous genes. Such functional equivalents will typically display a high degree of sequence identity with the endogenous gene to be inhibited (e. g. at least 70%, preferably at least 80%, and more preferably at least 90% identity). Accordingly, such functionally equivalent sequences will normally be able to hybridise under stringent hybridisation conditions (e.g. as described by Sambrook *et al,* 1989 Molecular Cloning. A Laboratory Manual, CSH, i.e. washing with 0.1xSSC, 0.5% SDS at 68°C) with (depending on orientation) either the -ve, or the +ve, strand of the naturally occurring endogenous plant gene whose expression is to be inhibited.

The invention further provides, in fourth aspect, a method of altering a potato plant or potato plant cell, the method comprising the steps of providing nucleic acid sequences; and introducing said sequences into the plant or cell, wherein the introduced sequences specifically inhibit the expression of GBSSI and at least one further starch synthase enzyme, each enzyme being involved in starch synthesis in the plant or cell. The method will typically be performed so as to produce a potato plant cell in accordance with the first aspect, or a potato plant in accordance with the second aspect of the invention defined previously.

Methods of introducing nucleic acid into plant cells or plants are well-known to those skilled in the art and do not require detailed elaboration. Any such suitable method (e.g. Agrobacterium-mediated transformation, protoplast fusion, "biolistic" transformation) may be employed. Conveniently the further starch synthase enzyme whose expression is inhibited by the method will comprise the SSIII and/or SSII genes (preferably both SSIII and SSII) of potato. If desired, the expression of other enzymes involved in starch synthesis may be inhibited instead of, or in addition to, inhibition of GBSSI, SSIII and/or SSII.

Performance of the method of the fourth aspect of the invention will generally lead to a change in the starch composition of the potato plant or plant cell.

Typically the invention may provide a potato plant which gives rise to altered starch having unexpected, extremely useful properties. Starch preparations may be produced from potato plants by means of a simple process: potato tubers are ground or milled or otherwise broken up, and the resulting starchy mass washed with water to remove cell debris and other contaminants, and the slurry then dried to significantly reduce the water content, leaving an easily-handled dried starch preparation. In particular, in one embodiment the invention provides a potato plant which gives rise to potato starch which, when in native form extracted from the plant, exhibits freeze-thaw stability such that a 1 %w/v aqueous suspension of the starch has an absorbance at 700nm wavelength of 1ss than 1.2 units (preferably less than 1.0 units) following 4 cycles of freezing overnight at -70°C and thawing for at least 2 hours at room temperature (i.e. at a temperature in the range 20-25°C). The invention also provides a potato plant which gives rise to potato starch which, when in native form extracted from the plant, exhibits freeze-thaw stability such that a 1 %w/v aqueous suspension of the starch has an absorbance at 700nm wavelength of less than 0.9 units (preferably less than 0.7 units) following 3 cycles of freezing at -70°C and thawing at room temperature as defined above. The invention further provides a potato plant which gives rise to potato starch which when in native form extracted from the plant, exhibits freeze-thaw stability such that a 1 %w/v aqueous suspension of the starch has an absorbance at 700nm wavelength of less than 0.7 units (preferably less than 0.5 units) following 2 cycles of freezing at -70°C and thawing at room temperature as defined above. The invention also provides a potato plant which gives rise to potato starch which, when in native form extracted from the plant, exhibits freeze/thaw stability such that a 1% w/v aqueous suspension of the starch has an absorbance at 700nm wavelength of less than 0.5 units, preferably less than 0.3 units, following 1 freeze/thaw cycle of freezing at -70°C overnight and thawing at room temperature for at least two hours.

Advantageously, the starch obtained from the potato plant of the invention will meet all of the separate absorbance criteria defined above after the requisite number of freeze-thaw cycles (e.g. have an absorbance of less than 0.5 units after 1 cycle, less than 0.7 units following 2 cycles; less than 0.9 following 3 cycles; and less than 1.2 units following 4 cycles).

(It will be readily apparent from the data presented in the Examples below, that starch obtained from the potato plant in accordance with the invention may display freeze-thaw stability (as judged by this method) well beyond four such freeze-thaw cycles, and that this number is selected purely for the purposes of illustrating the invention.

Absorbance values are one measure of the freeze-thaw stability of a starch. The inventors have also characterised the starch obtained from the potato plant of the invention by analysis of syneresis values following exposure to cycles of freezing and thawing, which are a measure of the strength of the gel formed by the starch.

In another embodiment the invention provides a potato plant in accordance with the second aspect which gives rise to potato starch which, when in native form extracted from the plant, exhibits freeze-thaw stability, such that a 5% w/v aqueous paste of the starch exibits less than 40 % syneresis (preferably less than 30%, more preferably less than 20%, and most preferably less than 10% syneresis) following 4 cycles of freezing at -70°C overnight and thawing at 22°C for 60 minutes, and then spinning at 8,000g for 10 minutes at 18°C. In a further embodiment the invention provides a potato plant which gives rise to potato starch which, when in native form extracted from the plant, exhibits freeze-thaw stability, such that a 5% w/v aqueous paste of the starch exhibits less than 30% syneresis (preferably less than 20%, and more preferably less than 10 % syneresis) following 3 cycles of freezing at -70°C overnight and thawing at 22°C for 60 minutes, and then spinning at 8,000g for 10 minutes at 18°C. Further, the invention provides a potato plant which gives rise to potato starch which, when in native form extracted from the plant, exhibits freeze-thaw stability, such that a 5% w/v aqueous paste of the starch exhibits less than 30% syneresis (preferably less than 20%, and more preferably less than 10% syneresis) following 2 cycles of freezing at -70°C overnight and thawing at 22°C for 60 minutes, and then spinning at 8,000g for 10 minutes at 18°C. Advantageously, the starch will meet all of the foregoing separate syneresis criteria after the requisite number of freeze-thaw cycles (e.g. less than 10% syneresis after 1 cycle; less than 20% after 2, less than 30 % after 3 and less than 40% after 4 cycles).

The inventors also tested the freeze-thaw stability of starches using a shorter experimental protocol.

Accordingly, in a further embodiment the invention provides a potato plant in accordance with the second aspect which gives rise to potato starch which, when in native form extracted from the plant, exhibits freeze-thaw stability, such that a 5% w/v aqueous paste of the starch exhibits less than 40% syneresis (preferably less than 30%, more preferably less than 20% syneresis) following 4 cycles of freezing at -70°C for 1 hour and thawing at 22°C for 10 minutes, and then spinning at 8,000g for 10 minutes at 18°C. In another embodiment the invention provides a potato plant which gives rise to potato starch which, when in native form extracted from the plant, exhibits freeze-thaw stability, such that a 5% w/v aqueous paste of the starch exhibits less than 40% syneresis (preferably less than 30%, more preferably less than 20%, and most preferably less than 10% syneresis) following 3 cycles of freezing at -70°C for 1 hour and thawing at 22°C for 10 minutes, and then spinning at 8,000g for 10 minutes at 18°C. The invention may also provide a potato plant which gives rise to potato starch which, when in native form extracted from the plant, exhibits freeze-thaw stability, such that a 5% w/v aqueous paste of the starch exhibits less than 30% syneresis (preferably less than 20%, more preferably less than 10% syneresis), following 2 cycles of freezing at -70°C for 1 hour and thawing at 22°C for 10 minutes, and then spinning at 8,000g for 10 minutes at 18°C. Advantageously, the starch will meet all the foregoing syneresis criteria after the requisite number of freeze-thaw cycles (e.g. less than 20% syneresis after 2 cycles; less than 30% after 3; less than 40% after 4 cycles).

Starch having any of the above properties, which is hereinafter generally referred to as "freeze-thaw stable starch", is extremely desirable for use in circumstances where compositions comprising starch are subjected to conditions in which at least one freeze-thaw cycle is likely to occur. In addition to conditions where freezing occurs, such starch may be useful in situations where compositions comprising starch are subjected to prolonged storage at low temperatures but where the temperature is insufficient to cause freezing of the composition.

In particular, starch as described above is useful in substances which are frozen, intentionally or otherwise, during production, distribution, storage or retail, and where thawing, intentionally or otherwise, may occur subsequent to freezing, or where the substances are exposed for long periods to storage at low (but not freezing) temperatures. Specific envisaged examples include use of starch compositions (comprising starch in accordance with the invention) as thickeners e.g. in foodstuffs or in industrial applications (e.g. paper-making; sizing or coating of surfaces) or in personal care products (e.g. cosmetics or other substances for treatment of the skin and/or hair).

Methods of making suitable starch compositions and incorporating the into foodstuffs and other substances will be apparent to those skilled in the art. Specific examples are given, for instance, in US 4,428,972; US 4,876,336; US 4,767,849 and EP-A-0796868.

Freeze-thaw stable starch exhibits freeze-thaw stability whilst in its native form (i.e. without having undergone physical, enzymatic or chemical modification *in vitro*), and thus avoids the need for expensive and inefficient *in vitro* modification in order to render the starch freeze-thaw stable. Nevertheless, the starch may be subjected to *in vitro* modification if desired, in order to enhance the freeze-thaw stability and/or modify other characteristics of the starch. Methods and processes for *in vitro* modification of starch are well known and include those, for example, disclosed in EP-A-0796868.

The invention also provides a potato plant giving rise to native starch having other useful properties, which are typically, but not necessarily, associated with freeze-thaw stability. Thus, in yet another embodiment the invention provides a potato plant which gives rise to starch which, when in native form extracted from the plant, has an apparent amylose content of less than 8% (preferably less than 6%, more preferably less than 4%, most preferably less than 3%), as determined by the method of Morrison & Laignelet (1983 Cereal Science 1, 9-20), and a viscosity onset temperature of less than 67°C, (preferably less than 65°C, more preferably less than 55°C, and most preferably less than 51°C) as determined by viscometric analysis of a 7.4% (w/v) aqueous suspension of the starch using a Rapid Visco Amylograph (RVA), Newport Scientific Series 4 instrument operating on the standard 1 heating and stirring protocol. Preferably such starch will be freeze-thaw stable and possess one or more of the attributes described above.

The method of Morrison & Laignelet, referred to above, is only one of a number of methods available for determination of the amylose content of a particular starch. Other methods include the potentiometric method of Shi et al, (1998 J. Cereal Sci. 27, 289-299) or determination of the amount of linear chains by gel permeation chromatography, as described below.

In another embodiment, the invention provides a potato plant which gives rise to potato starch which, when in native form extracted from the plant, has an apparent amylose content of less than 8% (preferably less than 6%, more preferably less than 4%, most preferably less than 3%) as determined by the method of Morrison & Laignelet (1983 Cereal Science 1, 9-20) and a ratio of fraction I to fraction II (explained below) short chain glucans of at least 60%, preferably at least 65%, more preferably 70% or more. Desirably such starch will also have one or more of the attributes described above.

In another embodiment, the invention provides a potato plant which gives rise to potato starch which, when in native form extracted from the plant, has an apparent amylose content of less than 8% (preferably less than 6%, more preferably less than 4%, most preferably less than 3%) as determined by the method of Morrison & Laignelet (1983) and, when analysed by differential scanning calorimetry using a Perkin Elmer DSC 7 instrument, a 10mg starch sample in aqueous mix of less than 25% starch w/v exhibits a gelatinization onset temperature of less than 67°C, more preferably less than 66°C, and most preferably less than 51°C. Conveniently such starch will also have one or more of the attributes discussed above.

The various aspects of the invention will now be further described by way of illustrative example and with reference to the accompanying drawings.

### Brief Description of the Drawings

The present invention may be more fully understood by reference to the following description, when read together with the accompanying drawings:
Figure 1 is a schematic diagram of the plant transformation vector pSJ152;
Figure 2 shows RVA analysis of starches from 17.21 transformed lines;
Figure 3 shows RVA analysis of starches from 17.29 transformed lines;
Figure 4 shows gel permeation chromatograph of starches from 17.21 transformed lines;
Figure 5 shows gel permeation chromatograph of starches from 17.29 transformed lines;
Figure 6 shows GPC analysis of debranched starches from 17.21 transformed lines;
Figure 7 shows GPC analysis of debranched starches from 17.29 transformed lines;
Figure 8 shows high performance anion exchange chromatrography (HPAEC) analysis of debranched starches from 17.21 transformed lines;
Figure 9 shows HPAEC analysis of debranched starches from 17.29 transformed lines.
Figure 10 shows the difference profile in chain length distribution for various starches;
Figures 11 and 21 illustrate freeze-thaw stability of various starches, as measured by absorbance values;
Figures 12 and 22 illustrate freeze-thaw stability of various starches, as measured by determination of % syneresis;
Figure 13 is a schematic representation of the vector pPOT3;
Figure 14 is a schematic representation of the plant transformation vector pSJ119;
Figures 15A and 15B are micrographs of starch granules;
Figure 16 shows RVA analysis of various starches;
Figure 17 shows the results of GPC analysis of various starches;
Figure 18 shows the results of GPC analysis of various starches following debranching with isoamylase;
Figure 19 shows the results of HPAEC analysis of various starches; and
Figure 20 shows the difference profile in chain length distribution for various starches.

### Detailed Description of the Invention

### EXAMPLE 1

### METHODS

### 1.1 Plant Material

Potato tubers (*Solanum tuberosum L.*) of cultivar Desiree were used. The tubers were grown in pots of soil based compost (25cm diameter) in a greenhouse with minimum temperature of 12°C and supplementary lighting in winter.

### 1.2 Construction of plant transformation vector

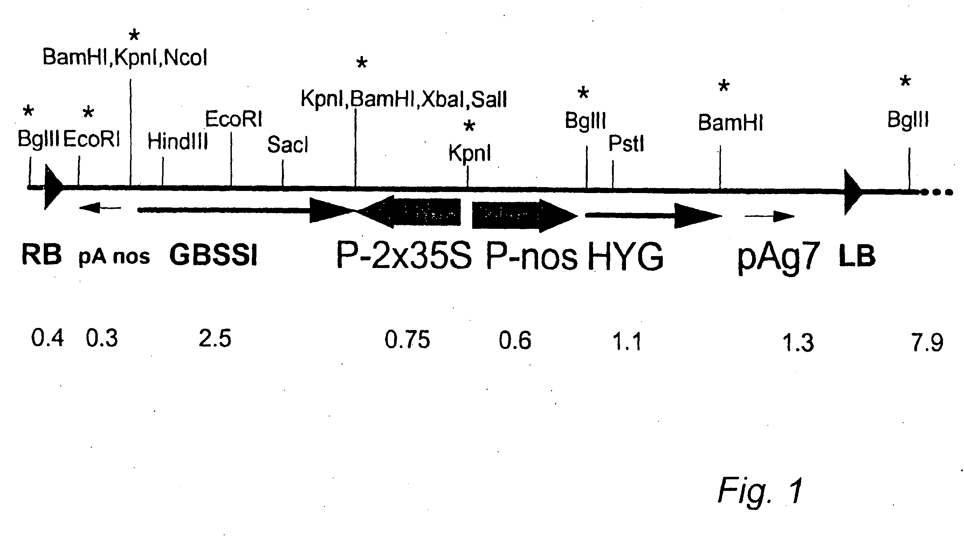

A full length 2465 bp cDNA for GBSS I (5' and 3' terminal sequences AGACCACAC... ...GTAAGGTAG equivalent to EMBL accession number X58453) was isolated from a potato tuber (cv. Desiree) cDNA library using the pea GBSSI cDNA as probe using standard techniques (Sambrook *et al* 1989). Following addition of linker sequences, the full length cDNA was cloned as a *Bam*HI fragment between the GBSS promoter and NOS poly(A) site in the plant transformation vector pGPTV-HYG (Becker et al 1992 Plant Mol. Biol. 20, 1195-1197). A map of the construct (pSJ152) is shown in Figure 1. In this figure the filled triangles represent the T-DNA borders (RB= right border and LB = left border), relevant restriction enzyme sites are shown above the black line with the approximate distances (in kilobases, kb) between sites marked by an asterisk shown underneath. Small arrows represent polyadenylation signals (pAnos = nopaline synthase, pAg7 = Agrobacterium gene 7), intermediate arrows denote protein coding regions (HYG = hygromycin resistance gene, GBSS = granule bound starch synthase) and the thick arrows indicate promoter regions (P-GBSS = granule bound starch synthase promoter and P-nos = nopaline synthase promoter).

### 1.3 Transformation of potato plants

Transformed potato plants cv. Desiree were produced by co-cultivation of explants with Agrobacterium tumefaciens LBA 4404 containing plant transformation vector plasmids. Two explant types were used, microtubers and leaf fragments, both produced from stock cultures, requiring different methods in culture. Stocks of plantlets were maintained of wild type untransformed Desiree or selected lines previously transformed with antisense genes coding for enzymes of the starch biosynthetic pathway by regular transfer of single node explants using Murashige and Skoog (MS) media (Murashige and Skoog. Physiol. Plant 15, 473-479 1962) solidified with 0.8 % agar with the sucrose concentration reduced from 3 % to 1%. Microtuber explants were produced by transferring nodes to the same medium with the sucrose concentration increased to 8% plus the addition of 2.5mg/l benzylaminopurine. All cultures were grown at 22°C with illumination from fluorescent tubes at 40µE m² for 16 hours except those to produce microtubers which were kept in darkness. The protocol for leaf explants is a modification of that published by Rocha-Sosa et al (1989 EMBO J. 8, 23-29).

Explants were initially precultured on the appropriate multiplication medium for two days before co-cultivation. Co-cultivation was for 10 minutes in an overnight culture of the Agrobacterium before explants were blotted on filter paper to remove excess bacteria. After blotting explants were transferred to filter paper on feeder layers and incubated in darkness at 22°C for two days. The feeder layers consisted of a 9 cm petri dish containing the appropriate multiplication media covered by 2 ml of stationary phase tobacco suspension culture cells which were in turn covered by two layers of filter paper.

After two days the explants were removed and washed in MS medium without sucrose or agar containing 500µg/ml cefotaxime, blotted on filter paper and transferred to multiplication medium containing 500µg/ml cefotaxime. After a further five days the explants were further transferred to the same medium containing the selection agent. Selection was effected by the use of the NPTII gene in the construct and addition of kanamycin in the tissue culture process or the HPTII gene with additions of hygromycin.

Tissue culture and selection using microtuber explants was performed as follows. Microtubers were taken from stock when they were sufficiently large and mature to use. This occurred after 6-8 weeks from transfer of nodes to the high sucrose medium when they weighed more than 30 mg and had started to change colour from white to pink. Stocks were used for several months after tuber initiation provided they remained dormant and no new shoots or stolons had emerged from the bud end. Each tuber was cut in half longitudinally through the terminal bud and the cut end put in contact with the medium. The multiplication medium (Z5) used contained the salts and vitamins of MS medium plus 3% sucrose, 0.2mg/l indole acetic acid, 5 mg/l zeatin and 0.8% agar. Kanamycin was added at 100mg/l when NPTII was used and hygromycin at 15mg/l when HPTII was used. The explants were transferred to fresh medium every two weeks. Explants had two transfers on medium Z5, followed by one transfer on the same medium except indole acetic acid and zeatin were replaced by 10mg/l gibberellic acid (medium MSG). At each transfer all shoots were removed and discarded. The final transfer was to hormone-free MS medium with the sucrose concentration reduced from 3 % to 1 %. Shoots were allowed to develop at this stage.

Tissue culture and selection using leaf explants was performed as follows. Expanded leaves were taken from stock cultures and cut into fragments comprising a half or a third of the leaf. The multiplication medium used contained the salts and vitamins of MS medium plus 1.6% glucose, 0.02 mg/l naphthyl acetic acid, 2mg/l zeatin riboside and 0.8% agar. Kanamycin was added at 100mg/l when NPTII was used and hygromycin at 5mg/l when HPTII was used. Cultures were transferred every two weeks to the same medium. After four transfers the cultures which were vitrified or showed poor shoot development were transferred to liquid MS medium containing 1.6% sucrose only.

Transformation was confirmed as follows. Shoots which developed from either tuber or leaf explants after the multiplication/organogenesis phase were transferred individually to hormone-free MS medium containing 1% sucrose and 100 mg/l kanamycin or 15mg/l hygromycin depending on the selection which was used. Only one shoot was taken from each explant unless the origin of separate shoots were well separated on the explant. Shoots which rooted on selection within two weeks were transfer to the same medium without selection and given an identifying number. When growth allowed sufficient tissue to be sampled, DNA was extracted from a few mgs of leaf and stem tissue of the culture using the method published by Edwards et al (1991 Nucleic Acids Research 19, (6) 1349) and transformation was confirmed by PCR using gene specific primers. Material was left in culture to allow individual shoots to be micropropagated. Positive shoots were micropropagated to be stored *in vitro* and also to provide planting material.

### 1.4 Growth of Transformed Lines

Five plants from each line, when 50-100mm high, were transferred to compost made up of 50% horticultural sand and 50% Levington F2 peat-based compost in 20 mm square modular pots and maintained at 20°C (day) and 15°C (night) in a growth room illuminated for 16 hours with high pressure sodium lamps at an illumination of 400µE m². After watering they were covered within a small propagator and shaded from illumination. After 7-10 days when new growth was apparent the shading was removed and the plants grown on. With an intermediate potting to 80 mm diameter pots, the five plants were finally grown to maturity together in a 250 mm diameter pot in Levington F2 compost under glass. Sixteen to eighteen weeks after transfer from culture when the foliage began to die down all the tubers were harvested. Representative samples were stored for analysis and possible regrowth whilst 150-200 g fresh weight was taken for starch extraction.

### 1.5 Starch Extraction and Analysis

Starch was extracted from washed potato tubers by dicing and passage through a Braun MP 75 centrifugal juicer. The juice was diluted with tap water to a volume of approximately 3L in a 5L conical flask. After 30 minutes the supernatant liquid was discarded and the remaining solids washed again with a further 3L of water. After a further 30 minutes the liquid was decanted and discarded. The slurry of solids was poured through a 500µm metal mesh filter which retained the bulk of the cell debris. The crude starch which passed through the filter was washed in the same manner until no protein foam remained and only white starch settled on the base of the flask below a clear supernatant liquid. The starch was harvested on filter paper using a Buchner funnel and washed on the funnel with two 500ml volumes of distilled water. Finally the starch was washed with 500ml of acetone and dried in a fume hood for approximately 30 minutes.

Detection of GBSS I (waxy) protein bound to the starch granule was performed as follows. Proteins were extracted from starch granules by boiling for 5 min in 2xSDS Laemmli sample buffer (600µl/20 mg starch) followed by one or two freeze thaw cycles (freeze on dry ice, thaw in water). Insoluble material was removed by spinning for 5 min at 15000g and 20-30µl of supernatant was analysed on a 7.5% SDS-PAGE gel and proteins were detected by silver staining.

The viscometric analysis of starches was performed with a Rapid Visco Analyser Series 4 instrument (Newport Scientific, Sydney Australia). For this 13 minute profile, 2g of starch was weighed into a sample cup and 25ml of water was added to give a final starch concentration of 7.4% (w/w) and the analysis was performed using the standard 1 stirring and heating protocol according to the manufacturer:
Standard 1 profile;
starting temperature 50°C;
from 1 min to 4 min 45 sec temperature rises at a rate of 1°C/5 seconds to 95°C
from 4 min 45 sec to 7 min 15 sec temperature holds at 95°C
from 7 min 15 sec to 11 min temperature lowers at a rate of 1°C/5 seconds to 50°C
from 11 min to 13 min temperature holds at 50°C. The sample was stirred with a paddle speed of 960 rev/min for 10 seconds to mix the sample, and then the speed was reduced to 160 rev/min for the remainder of run.

Peak onset and pasting values (in centipoise, cP) were automatically determined using the manufacturer's software. Differential scanning calorimetry was performed essentially as described in WO 95/26407 except that larger samples (approx 10 mg of starch) and larger sized aluminium pans were used. Duplicate samples were measured where appropriate and standard errors calculated. Microscopic examination of starch granules was performed by mixing a small amount of starch with Lugol's iodine solution (1% I₂/KI), diluted 1:1 (v/v) with water. Amylose content was performed by the iodine colorimetric method of Morrison and Laignelet (1983 Cereal Sci. 1, 9-20).

### 1.6 High Performance Anion Exchange Chromatography (HPAEC)

Chain-length distribution was determined by HPAEC with pulsed amperometric detection ("PAD") (Dionex Corp., Sunnyvale, CA). A Dionex Carbopac PA-100 column (4x25mm) was used with a Carbopac PA Guard column (3x25 mm). The potential and time settings on the PAD cell were: E₁ = 0.10V (t₁ = 480MS); E2 = 0.60V (t₂=120MS); E3 = -0.80V (t₃= 300ms). The eluent A was 150 mM sodium hydroxide solution which was prepared by dilution of carbonate-free sodium hydroxide in deionised water. The eluent B was 150 mM sodium hydroxide solution containing 500 mM sodium acetate. The gradient programme starts with 85% eluent A and 15% eluent B and ends with 100% eluent B as detailed below, followed by a ten minute flush with the starting gradient to prepare the column for re-use.

| **Time** | **%A** | **%B** |
|---|---|---|
| | 85 | 15 |
| 0.0 | 85 | 15 |
| 0.1 | 85 | 15 |
| 0.4 | 65 | 35 |
| 20.0 | 50 | 50 |
| 35.0 | 40 | 60 |
| 50.0 | 30 | 70 |
| 75.0 | 25 | 75 |
| 85.0 | 0 | 100 |
| 87.0 | 85 | 15 |
| 95.0 | 85 | 15 |
| 105.0 | | |

To prepare the samples, each starch sample (20mg) was weighed into a 10ml vial; 2 ml of 90% DMSO (dimethyl sulfoxide) (DMSO:water = 9:1, volume/volume) was added, mixed with a magnetic string bar, and heated in a boiling water bath for 5 minutes. Then, 7ml of water was added and mixed. After the mixture was cooled to room temperature, 1ml of 150mM sodium hydroxide was added, mixed and 1ml of the reaulting solution was run by the Dionex.

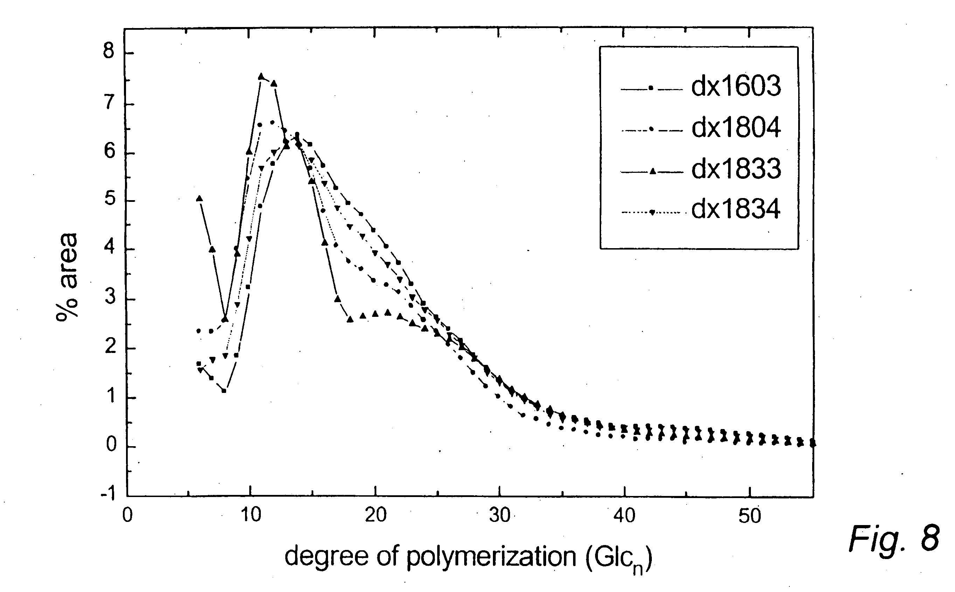

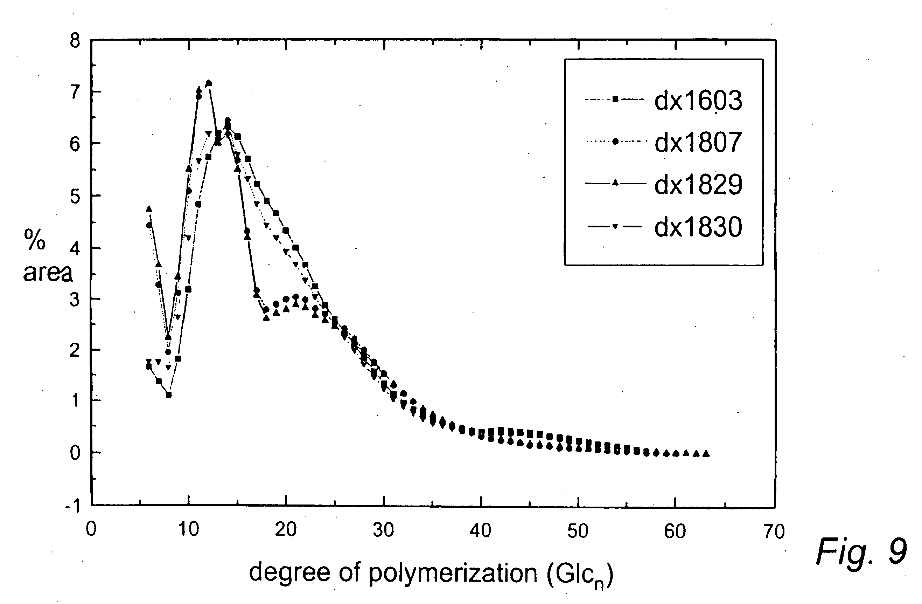

All separations were carried out at ambient temperature at a flow rate of 1 ml/minute The data (see Figures 8 and 9) are presented as a percentage of total peak area without regard for changes in detector response, which varies with chain length (the detector being less sensitive to chains of short length).

### 1.7 Gel Permeation Chromatography

A Water Associates (Milford, MA) GPC-150 model with refractive index (RI) detector was used to determine molecular weight distribution. Two PL gel columns (10⁵ and 10³) made of highly crosslinked spherical polystryene/divinylbenzene, were obtained from Polymer :laboratories (Amherst, MA) and connected in sequence. Dextrans from American Polymer Standards (Mentor, Ohio) were used as size (molecular weight, Mw) standards. Columns were run at 80 °C at a flow rate of 1 ml/min with a mobile phase of dimethyl sulfoxide in 5 mM sodium nitrate with a sample concentration of 1.25 mg/ml and an injection volume of 300 µl.

### 1.8 Freeze-thaw stability determination

Freeze-thaw stability of starches was assessed after heating a 1% w/v starch suspension in water (10 ml in a tightly capped 15 ml tube) in a boiling water bath for 15 minutes with frequent agitation and cooling to room temperature (20-25°C). The absorbance at 700 nm (1cm path length, water as blank) of the resultant pastes was determined before and after repeated freeze thaw cycles. Starch pastes were frozen by placing in a -70°C freezer for a minimum of 16 hours (overnight) and up to a maximum of 58 hours (over a weekend). Thawing was allowed to occur at room temperature for 2-3 hours and then the absorbance at 700 nm was determined and the sample then placed back in the freezer.

### 1.9 RESULTS

### 1.9.1 Generation of potato plants with combined reductions in SSII, SSIII and GBSSI.

Two transgenic lines (17.21 and 17.29) showing strong, almost complete, inhibition of SSII and SSIII as assayed by zymogram analysis were chosen to be retransformed with a waxy antisense construct (pSJ152, fig. 1). Line 17.29 has been described in detail previously (Edwards et al 1999). It appeared that line 17.29 had a more severe phenotype than line 17.21, in that starch from plants of line 17.29 gelatinised at a lower temperature as judged by DSC analysis (Table 1). The starch granule morphology also differed between the two lines, in that starch from line 17.21 had severely cracked granules whereas starch granules from line 17.29 appeared to have more sunken centres (data not shown).

Transformed plants were selected on hygromycin and additionally screened by PCR for the presence of the waxy antisense T-DNA. Plants were identified by a unique number; 1801-1805 were regrowths of line 17.21, plants 1806-1809 were regrowths of line 17.29, and 1810 onwards were doubly transformed plants. Twenty-four plants of line 17.21 and nineteen of line 17.29 were generated and grown with controls to maturity in the greenhouse, starch was extracted and analysed. Analysis of proteins bound to the starch granule showed that seven of the 17.21 derived lines (1810,1812,1813,1833,1834,1842 and 1843) and two of the 17.29 derived lines (1829 and 1830) had complete or almost complete loss of GBSSI. These lines were studied further. Transgenic plants in which the expression of SSII and SSIII was inhibited are referred to as "SSII/III plant", and lines established from such plants are correspondingly referred to "SSII/III lines". Similarly, plants and lines in which GBSSI, SSII and SSIII were all inhibited are referred to as "GBSSI/SSII/III" plants and lines respectively. Starches from these plants may be referred to as SSII/III starch and GBSSI/SSII/III starch respectively. Plants in a particular line are genetically identical products of a particular transformation or recombination event.

### 1.9.2 Granule morphology and starch composition

The apparent amylose content of the starches was determined colourimetrically by iodine staining. Starch from the SSII/III lines had amylose contents of 32-33% i.e. similar to wild type levels (data not shown), whereas the starch from the lines that, in addition, had reduced levels of GBSSI had much lower apparent amylose levels, in most lines about 9-11 % amylose (Table 1). However two lines (1829 and 1833, derived from 17.29 and 17.21 respectively) showed much lower apparent amylose contents (less than 3%). The waxy (i.e. very low amylose) nature of these starches was confirmed by microscopic examination of iodine stained granules which showed that granules from all of these lines were predominantly red with small blue cores at the centre, in contrast to the wild type and control lines (17.21 and 17.29) which stained uniformly blue (data not shown). The granule morphology of these waxy lines had changed compared to the SSII/III control lines in that the deep cracks or fissures and sunken centres had disappeared and the granules now had more angular edges. In addition, there appeared to be many more smaller granules which also had angular edges and these were often found as small aggregates or in association with larger granules (data not shown).

| Code | RVA DATA | | | | | DSC DATA | | | | | | AMYLOSE | |
|---|---|---|---|---|---|---|---|---|---|---|---|---|---|
| | Onset | Peak | Peak | Pasting | Setback | Peak | s.e. | delta H | s.e. | Onset | s.c. | % | s.d |
| | (°C) | (°C) | (cP) | (cP) | (cP) | (°C) | | (J.g) | | (°C) | | | |
| 1603 | 67.8 | 89.6 | 4488 | 2483 | 3091 | 68.18 | 0.22 | 22.35 | 0.04 | 63.97 | 0.12 | | |
| J117.21a | 62.3 | 84.0 | 2242 | 1478 | 2160 | 59.97 | 0.18 | 20.69 | 0.76 | 51.66 | 0.17 | | |
| J117.21b | 61.5 | 87.3 | 2191 | 1415 | 2152 | 5962 | 0.28 | 19.21 | 0.20 | 51.43 | 0.08 | | |
| J117.29a | 62.1 | 95.1 | 1756 | 1513 | 2455 | 54.54 | 0.02 | 16.10 | 0.01 | 47.65 | 0.01 | | |
| J117.29b | 62.2 | 95.1 | 1801 | 15.65 | 2482 | 54.74 | 0.30 | 16.50 | 0.07 | 48.35 | 0.13 | | |
| 1803(21) | 63.0 | 86.5 | 2230 | 1535 | 2347 | 60.55 | | 18.85 | | 51.53 | | 33.54 | 0.38 |
| 1804(21) | 63.0 | 84.0 | 2274 | 1505 | 2294 | 60.24 | | 18.47 | | 51.37 | | | |
| 1807(29) | 62.2 | 94.6 | 1669 | 1440 | 2250 | 54.98 | | 15.88 | | 47.44 | | 31.91 | 0.43 |
| 1808(29) | 62.2 | 95.0 | 1718 | 1490 | 2506 | 55.38 | | 15.30 | | 47.24 | | | |
| 1810(21) | 68.7 | 74.3 | 6397 | 2273 | 2499 | 68.91 | | 22.00 | | 64.98 | | 16.73 | 0.51 |
| 1812(21) | 66.2 | 81.6 | 4153 | 2176 | 2074 | 68.71 | | 19.43 | | 59.22 | | 9.55 | 0.49 |
| 1813(21) | 65.4 | 79.1 | 4275 | 2124 | 2132 | 68.81 | | 19.77 | | 59.98 | | 9.77 | 0.19 |
| 1829(29) | 50.3 | 70.2 | 3110 | 1330 | 1688 | 53.22 | 0.31 | 17.80 | 0.28 | 48.63 | 0.28 | 2.67 | 0.29 |
| 1830(29) | 67.8 | 72.0 | 6331 | 1844 | 2011 | 68.53 | 0.00 | 22.35 | 0.26 | 65.00 | 0.07 | 11.04 | 0.87 |
| 1833(21) | 50.1 | 63.7 | 3099 | 1409 | 1543 | 54.29 | 0.07 | 18.57 | 1.19 | 49.46 | 0.01 | 2.8 | 0.21 |
| 1834(21) | 68.7 | 73.5 | 5543 | 2012 2 | 2032 | 69.67 | 0.21 | 21.87 | 0.26 | 65.62 | 0.06 | 11.29 | 2.79 |
| 1847(21) | 66.2 | 72.6 | 4800 | 1691 | 1738 | 67.59 | | 20.35 | | 63.20 | | 9.65 | 1.78 |

### 1.9.3 Effects on gelatinisation behaviour of starch

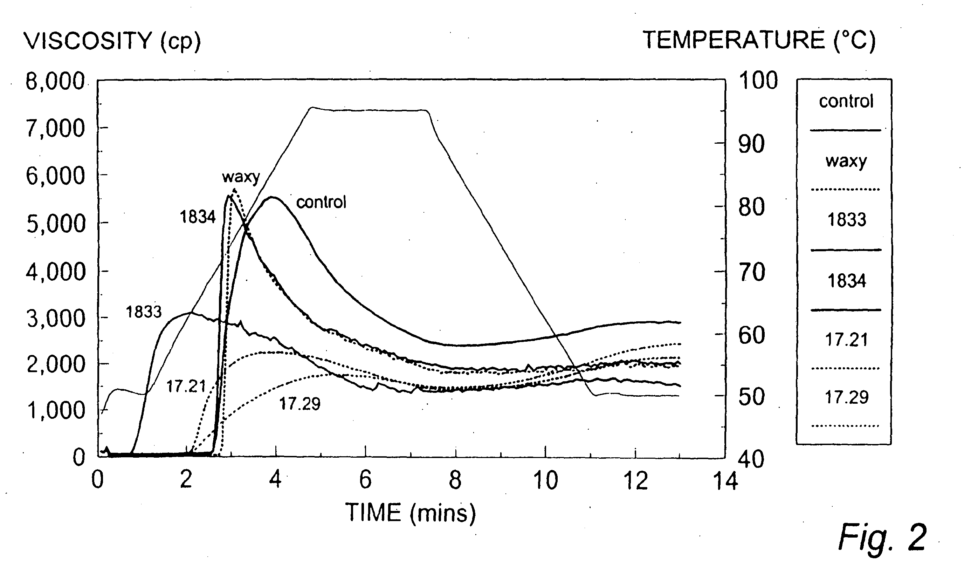

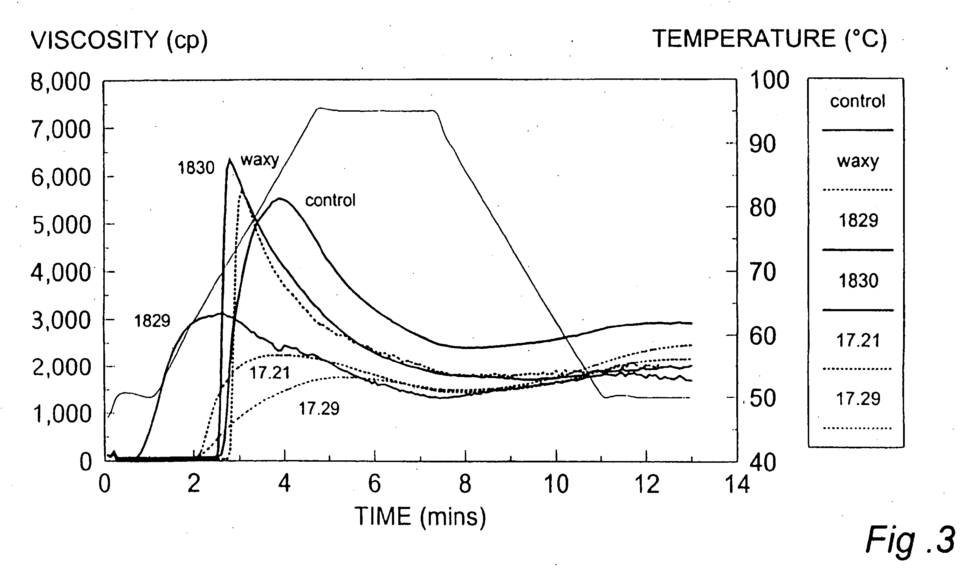

The physical properties of these starches was analysed by viscometric analysis (RVA) and by differential scanning calorimetry (DSC). These data are summarised in Table 1, and in Figures 2 and 3. Figures 2 and 3 are viscoamylographs, with the viscosity of the starch suspensions shown in centipoise (left hand scale) at various temperatures (in °C, right hand scale). The temperature profile is indicated by the thin linear plot. The behaviour of starch from wild type control plants is shown by the thick solid curve Starches from the various mutant lines are indicated by reference numerals adjacent to the curves.

As was shown previously (Edwards et al 1999), in RVA the SSII/III starches start to swell at much lower temperatures than the wild-type control (62°C vs 68°C) and the peak viscosity is also much lower. Line 17.29 starch consistently showed lower peak viscosities than line 17.21 starch. The starch from regrowths of these lines (e.g. 1803,1807) showed identical viscosity profiles (data not shown) indicating that these lines are phenotypically stable. It should be noted that the viscosity profiles are however affected quite dramatically by the temperature/time profile of the RVA analysis. The profile employed here is the manufacturer's standard quick assay (Standard 1) (14 min) and analysis of these starches using a longer, slower profile (90 min) showed that these starches actually swell at much lower temperatures (Edwards et al 1999). The onset and peak temperatures of gelatinisation as determined by DSC analysis of starch from the line 17.21, were approximately 4-5°C higher than for starch from line 17.29 (Table 1). In addition the enthalpy of gelatinisation of 17.21 starch was also higher suggesting that starch from line 17.29 had a reduced granule organisation compared to that from line 17.21.

Inhibition of GBSSI in the SSII/III lines dramatically altered the swelling properties of the starches. Two classes could be discerned and examples of these are shown in Figures 2 and 3. Many of the GBSSI/SSII/III lines (representative examples shown in Figures 2 and 3 are lines 1830 and 1834) produced starch with swelling characteristics very similar to waxy potato starch, in that the onset temperature was slightly increased compared to wild type and with sharp peak and high maximum peak viscosities. However the two GBSSI/SSII/III lines which had the lowest amylose levels (1829 and 1833, derived from lines 17.29 and 17.21 respectively) showed very low onset temperatures (50°C) with low peak viscosities compared to the wild type control. Analysis of these starches by DSC confirmed that these two lines were dramatically different from the other waxy lines. The gelatinisation onset of 1829 and 1833 starches was very low, (48 to 49°C) similar to the parent SSII/III lines (17.21,17.29 and 1803-1808), whereas the other lines showed much higher onsets of up to more than 65°C. The gelatinisation enthalpy of line 1833 was similar to the parent line whereas that of 1829 was slightly higher.

### 1.9.4 Molecular characterisation of starches

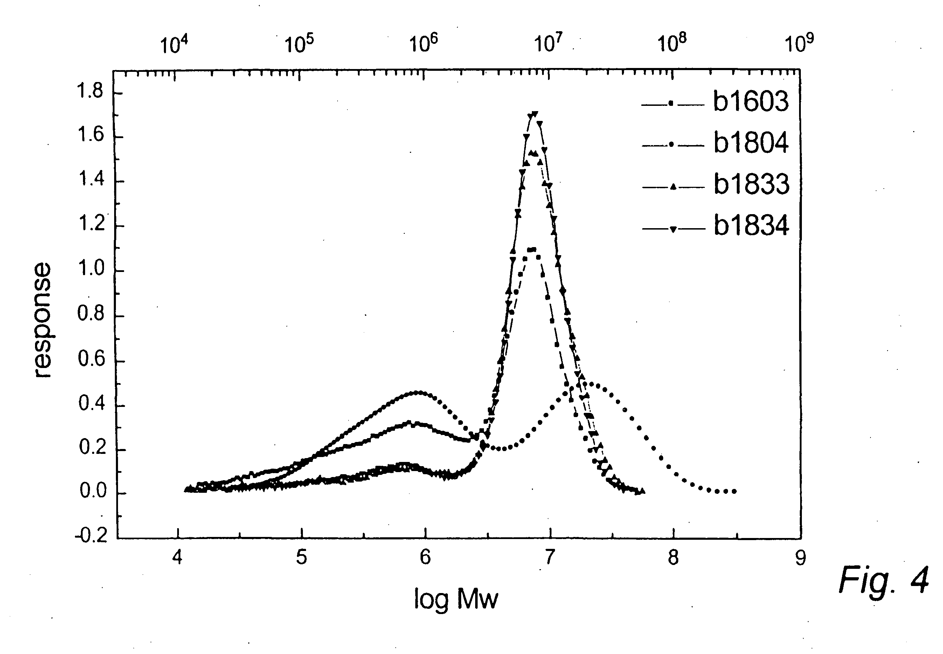

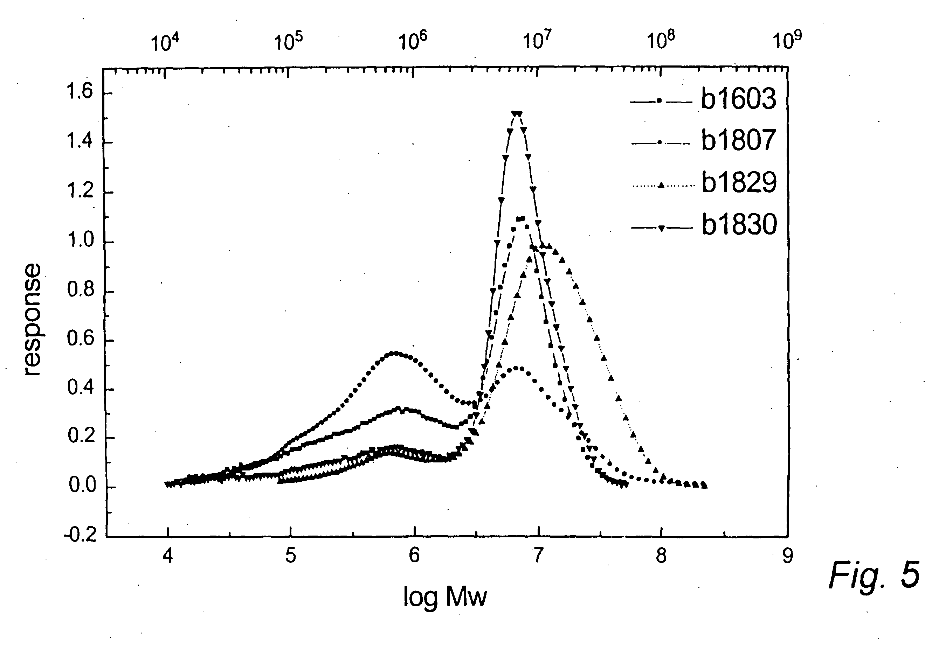

The molecular structure of the starches was further analysed. When control starch (labelled as 1603) was solubilised in DMSO and separated by gel permeation chromatography (GPC), two major fractions were resolved (Figure 4). Figure 4 is a graph of detector response (arbitary units) against log Mw, and shows the results of GPC analysis of starch from lines 1603 (control, wild type, square symbols), 1804 (SSII/III, circle symbols), 1833, and 1834 (GBSSI/SSII/III, upward and downward pointing triangles respectively). Figure 5 is a graph similar to Figure 4 and shows the results of GPC analysis for starch from lines 1603 (wild type control, square symbols), 1807 (SSII/III, circle symbols), and 1829, 1830 (GBSSI/SSII/III, upward and downward pointing triangles respectively).

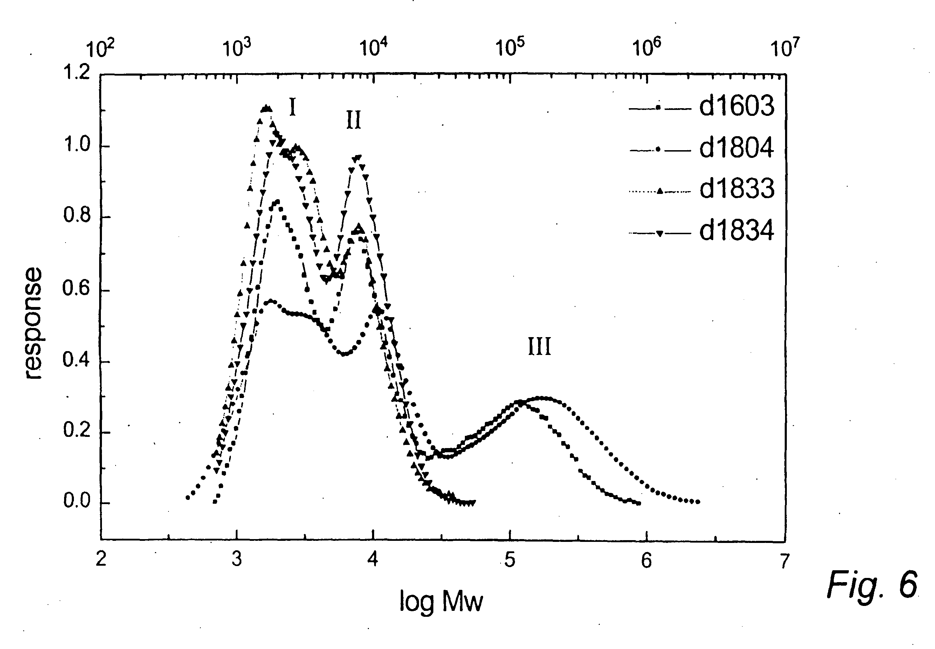

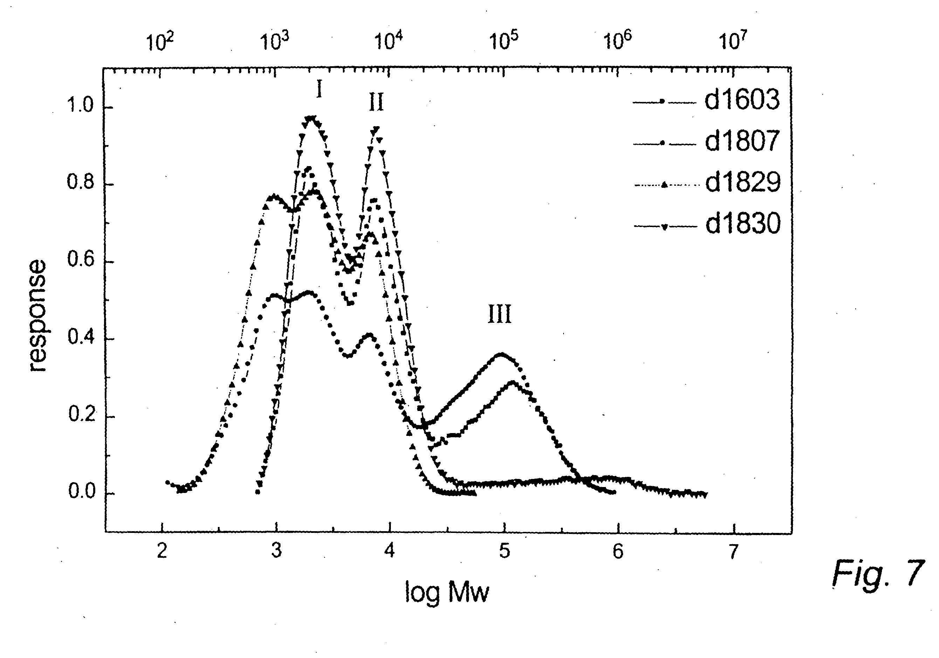

The major component eluted as a sharp symmetrical peak with a dextran-equivalent molecular size of about 10⁷. This peak contained the amylopectin fraction as it completely disappeared following debranching of the starch with isoamylase (see Figures 6 and 7). The second, amylose containing, fraction eluted as a broader, flatter peak with a molecular size range of 10⁵-10⁶. Not all of this material is amylose as integration of the area under this peak estimated that it represented 40 % of the total glucan whereas the amylose content determined by iodine binding was only about 30%. A significant amount of material with a molecular size of approximately 1x10⁶ is derived from amylopectin as it is also found in other waxy potato starches (data not shown). As has been shown previously (WO 99/66050) the SSII/III starch composition is greatly altered: lines 1804 and 1807 show a much decreased amylopectin fraction and an increased proportion of amylose. When GBSS I expression is reduced in the SSII/III lines the structure of the starch is further modified. The amylose-containing peak is nearly absent and the amylopectin fraction more closely resembles that of the wild type control (cf. 1833, 1834 & 1829, 1830 with 1603, Figs. 4 and 5).

A more detailed picture of the changes occurring in the amylopectin structure was obtained when the debranched starches (following treatment with isoamylase) were analysed by GPC (Fig. 6 and 7). Figures 6 and 7 are graphs of response (arbitary units) against log Mw, showing the results of GPC analysis of starch following debranching. Figure 6 shows results for starch from lines 1603 (control, square symbols), 1804 (round symbols), 1833 and 1834 (upward and downward pointing triangles respectively). Figure 7 shows results for starch from lines 1603 (control, square symbols), 1807 (round symbols), 1829 and 1830 (upward and downward pointing triangles respectively).

After debranching with isoamylase, wild type starch (1603) separates into three fractions designated as fraction I, II, and III in order of increasing size. The short glucan chains in fractions I and II are derived mainly from amylopectin whereas the long glucan chains in fraction III are mainly derived from amylose. Integration of the area under these peaks was used to calculate the proportion of material in each fraction. Boundaries of the fractions were identified as the minimum of the curves at approximate Mw of 5,000 and 20-30,000.

In the 1603 control fractions I, II and III represent 41 %, 35 % and 24% respectively of the total starch present. In this starch glucan chains in fraction I made up about 54% of the short chains ie I/(I+II) and this was consistently found in several control starches (from greenhouse grown material) in addition to 1603 (Table 2). In the SSII/III lines (1804, 1807) the proportion of fraction I had increased compared to the control (see Table 2 below). In line 1804 fraction I had increased to 61 % of the total short chains compared to 54% in the control, and line 1807 appeared to be more modified as fraction I had further increased to over 71 % of the total of short chains. The profile of fraction I in the SSII/III starches was also different from the control as a shoulder was evident in 1804 and this was resolved into a second distinct peak in line 1807. Starch from the 17.29 derived line (1807) again appeared to be more modified than that from 17.21 derived line (1804) in that there were also more glucan chains of less than molecular size 10³ in fraction 1. The proportion of amylose (fraction III) in the SSII/III starches had also increased to 29% and 33% (line 1804 and 1807 respectively) compared to 23% in the control.

In the GBSSI/SSII/III starches there was a clear difference between the lowest amylose-content lines (1829,1833) and the others (1830,1834). The former starches had branch chain profiles very similar to the corresponding original SSII/III starches (1807 and 1804 respectively) and fraction 1 made up more than 70% of the short chains, whereas in starch from 1830 and 1834 fraction I made up less than 56% of the short chains (Table 2) and the profiles looked more similar to wild type controls. However all the GBSSI/SSII/III starches clearly had very little amylose as evidenced by absence of linear chains in fraction III (Figs 6 and 7).

**Table 2**

| **line** | **Ratio I/(I+II)** | **Ratio III/(I+II+III)** |
|---|---|---|
| 1603 | 0.543 | 0.237 |
| Control 1 | 0.537 | 0.223 |
| Control 2 | 0.538 | 0.234 |
| 1804 (SSII/III) | 0.610 | 0.290 |
| 1833 (GBSSI/SSII/III) | 0.702 | 0.0 |
| 1834 (GBSSI/SSII/III) | 0.559 | 0.0 |
| 1807 (SSII/III) | 0.715 | 0.333 |
| 1829 (GBSSI/SSII/III) | 0.721 | 0.0 |
| 1830 (GBSSI/SSII/III) | 0.544 | 0.055 |

The debranched starches were also analysed by high performance anion exchange chromatography (HPAEC), using dionex columns, in order to determine what changes had occurred in the individual chain lengths (Figs 8 and 9). Figures 8 and 9 are graphs of % area under each peak (for each dp value: dp = degree of polymerisation) against degree of polymerization (as number of glucose residues), showing results of HPAEC analysis of the debranched starch. Figure 8 shows results for starch from lines 1603 (control, square symbols), 1804 (circle symbols), 1833 and 1834 (upward and downward pointing triangles, respectively). Figure 9 shows results for starch from lines 1603 (control. square symbols), 1807 (circle symbols), 1829 and 1830 (upward and downward pointing triangles, respectively).

In this analysis, chain lengths from dp 6 up to dp 55 were individually resolved and the abundance of each chain is expressed as a percentage of the total area. The control starch shows a typical chain length distribution with a sharp peak at dp 13-14 followed by a broad tail with very few chains with dp > 35. This profile is very reproducible and repeated analyses can be overlayed almost exactly so any deviations from this profile are significant.

The SSII/III starches (1804 and 1807) showed an increase in proportion of short chains with a peak at dp 11-12 and a decrease in the proportion of longer chains with a dp of 16-26. Again, starch derived from line 17.29 (1807) had a more severe phenotype than 17.21 (1804) since the former starch showed a greater increase in short chains (especially of dp 6-8) and a greater decrease in the longer chains (dp 16-26) than the latter. In the GBSSI/SSII/III starches there was also a clear difference between the lowest amylose lines (1829,1833) and the others (1830,1834). The former starches had a chain length distribution very similar to the most modified SSII/III starch (1807) i.e. a large increase in very short chains (dp 6-8) and a large decrease in longer chains (dp 16-26), whereas the starch from 1830 and 1834 had a chain length distribution similar to wild type.

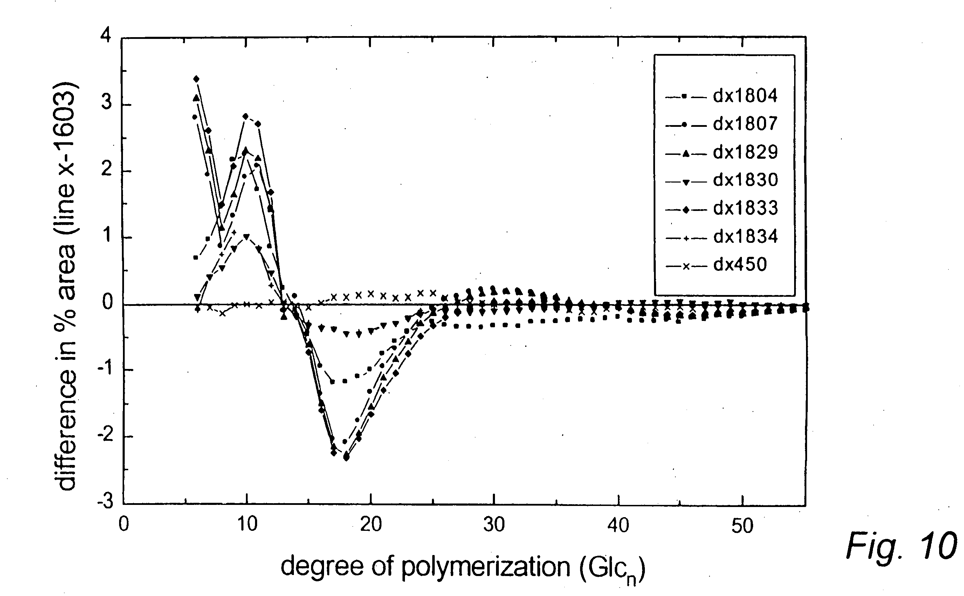

These changes can be more clearly seen when the data are plotted as a difference in chain length abundance from control (Fig. 10). Figure 10 is a graph of difference in % area (calculated as % area line X - % area line 1603) against degree of polymerization (number of glucose residues), and shows the results for starch from lines 1804, 1807, 1829, 1830, 1833, 1834 and an additional control line (labelled as 450). (1804 - square symbols; 1807 - round symbols; 1829 - upward pointing triangles; 1830 - downward pointing triangles; 1833 - lozenge symbols; 1834 - vertical cross symbols; 450 - diagonal cross symbols). As stated before, the reproducibility of the data is very good as can be seen from the difference profile of another control starch (line 450) which is very close to the origin across the entire distribution.

### 1.9.5 Effects on freeze-thaw stability of starch

### Absorbance Method

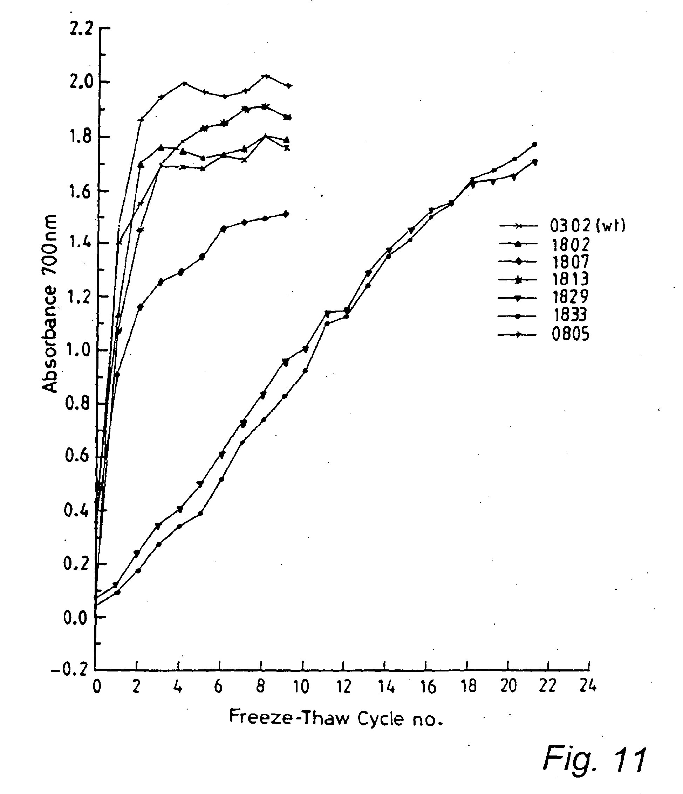

The freeze-thaw stability of the starches was assessed by following the increase in absorbance at 700 nm of starch pastes after freeze-thaw cycling. The absorbance measurements were used as a simple index of the consequences of the underlying molecular changes which take place during retrogradation. Higher values indicate more association between molecules whereas low values indicate little association or less retrogradation. Figure 11 shows the results from a typical experiment. Figure 11 is a graph of Absorbance at 700nm against Number of freeze-thaw cycles, and shows the results for analysis of freeze-thaw stability for starch 0302 (wild type control, diagonal cross symbols) and starch from lines 1802 (upward-pointing triangle symbols), 1807 (lozenge symbols), 1813 (asterisk symbols), 1829 (inverted, downward-pointing triangle symbols), 1833 (circle symbols) and 0805 (waxy starch, cross symbols).

All freshly prepared starch pastes had a clear appearance. However, the waxy starches had initial absorbances of less than 0.1 whereas all the amylose containing starches had significantly higher absorbances of between 0.3 and 0.4. After one freeze thaw cycle the waxy (0805), SSII/III (1802) and wild type control starch (0302) showed a rapid rise in absorbance which reached a maximum after 3-4 cycles. The SSII/III starch (1807) also showed a rapid increase initially but this was slightly slower than the other starches and the absorbance continued to increase after 3-4 cycles and reached a plateau between 7-9 cycles. As was apparent from the molecular characterisation of the starches. the 17.29 derived starch (1807) showed a more severe phenotype than the 17.21 derived starch (1802) in that the rate and the overall increase in absorbance was less, indicating that the freeze-thaw stability of this starch was greater. Starch from plants of line 1813 did not show any significant improvement in freeze-thaw stability compared to the SSII/III (1802) control. As described earlier this starch was one of the GBSSI/SSII/III starches that had an apparent amylose content of about between 9-11% and a chain length distribution as assayed by dionex which was very similar to 1834 (data not shown). In contrast, starch from the two GBSSI/SSII/III lines 1829 and 1833 which showed more extreme chain length distributions and had an amylose content of less than 3%, showed very high freeze thaw stability. The initial increase in absorbance after one freeze-thaw cycle was very small and subsequently increased in a linear fashion reaching a similar final absorbance as the control starches only after 21 cycles.

### Syneresis Method

The freeze-thaw stability of starches was further evaluated by measuring the amount of syneresis from starch pastes after centrifugation following a number of freezing and thawing cycles. Briefly, a 5% starch paste was made by weighing 0.75 g starch into a screw top MacConkey bottle and adding 15 ml of distilled water. The starch was dissolved by heating in a boiling water bath for 20 minutes with frequent shaking during the first 5 mins to ensure a uniform gel. After cooling to room temperature, aliquots of approximately 0.5 ml were distributed into preweighed 1.5 ml Eppendorf centrifuge rubes and the exact weight of starch paste in each tube (value x) was determined by reweighing the tubes. All tubes were frozen overnight at -70 °C and then thawed by placing in a 22°C water bath for 60 mins. For each starch paste, six tubes were spun for 10 minutes at 8000xg at 18°C immediately after which the tubes were drained by inverting onto an absorbent surface with gentle tapping to remove all the water (decomposition of the gel releasing water). After 10 minutes the inside of the tubes was carefully wiped to remove all remaining water making sure that the surface of the gel was not disturbed. The tubes were then reweighed to determine the mass of starch paste remaining in the tube (value y) and hence the mass of sample decanted (value x - value y). Syneresis is defined as the mass of starch sample decanted, expressed as a percentage of the original mass of starch paste (w/w). For example, a 0.5 gm sample showing 60% syneresis would decant a mass of 0.3 gms. The remaining tubes were placed back at -70°C for further freeze-thaw cycles and measurement.

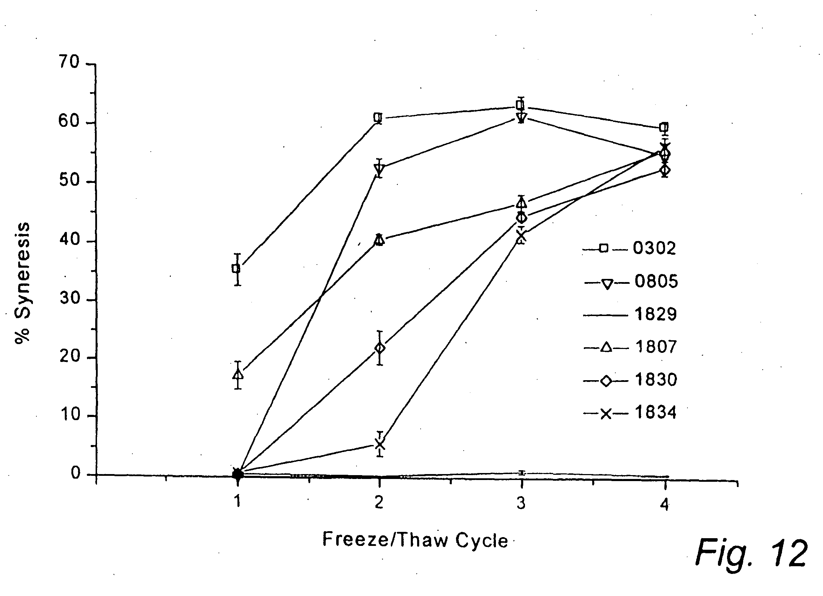

The results are shown in Figure 12, which is a graph of % syneresis against number of freeze/thaw cycles. The legend to the figure is as follows: 0302 (wild type) - square symbols; 0805 - downward pointing triangles; 1829 - line only, no symbols; 1807 - upward pointing triangles; 1830 - lozenge symbols; 1834 - cross symbols.

The wild type control starch was not freeze-thaw stable as after only one cycle approximately 35% syneresis was observed and this increased to 60% after two or more cycles. The SSII/III starch from line 1807 showed only slightly improved freeze-thaw stability since only 17% syneresis was observed after one cycle and this increased linearly with further cycles reaching the same level as the control after 4 cycles. All the waxy starches (0805, 1829,1830 and 1834) were freeze-thaw stable after one cycle as no syneresis was observed. However, the waxy starch 0805 was only freeze-thaw stable for one cycle as after two cycles this starch showed as much syneresis as the wild type control. The GBSSI/SSII/III lines 1830 and 1834 showed better freeze-thaw stability than the 0805 starch as only slight syneresis was observed after two cycles but this increased to control levels after four cycles. The GBSSI/SSII/III line 1829 showed complete freeze-thaw stability as no syneresis was observed during the four freeze-thaw cycles of the experiment.

### Example 2

### 2.1 Methods: Construction of plant transformation vectors.

### 2.1.1 pPOT3 (antisense GBSS I)

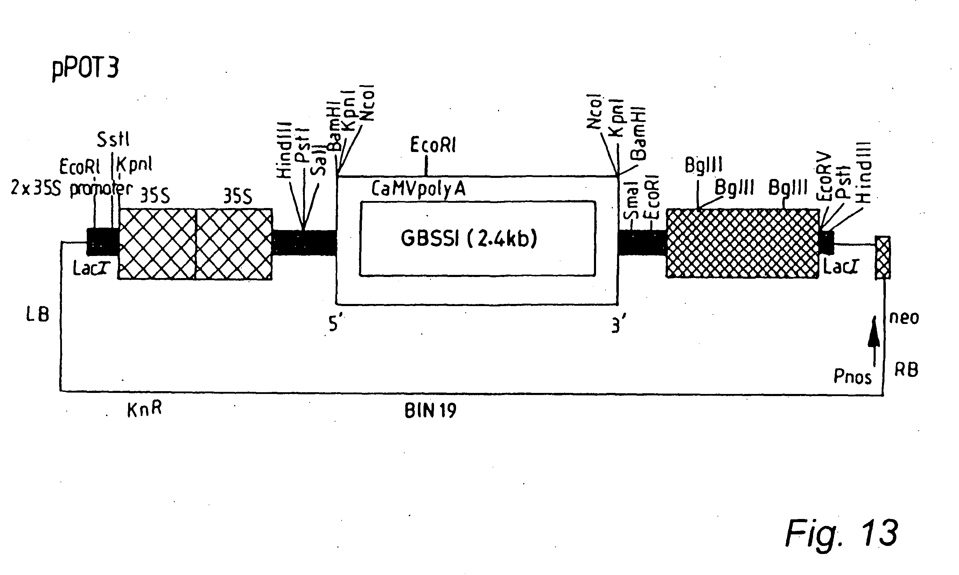

A full length 2465 bp cDNA for GBSS I was obtained as described at Example 1b above. Following addition of linker sequences, the full length cDNA was cloned as a *Bam*HI fragment in an antisense orientation between the cauliflower mosaic virus double 35S promoter and cauliflower mosaic virus terminator in pJIT60 (Guerineau and Mullineaux, 1993 Plant transformation and expression vectors. In Plant Molecular Biology Labfax (Croy, R.R.D., ed) Oxford, UK: Bios Scientific Publishers pp. 121-148) producing plasmid pPOT2. The *Xho*I-partial *Sst*I fragment from pPOT2, encompassing the promoter, antisense cDNA and terminator was ligated between the *Sal*I/*Sst*I sites of the plant transformation vector pBIN19 (Bevan, 1984 Nucl. Acids Res. 12, 8711-8721), resulting in plasmid pPOT3 (Fig 13). In Figure 13, the GBSSI sequence is shown by the white box (with border). The box to the right represents the double 35S promoter, and the box to the right with fine shading depicts the CaMV polyadenylation signal.

### 2.1.2 pRAT4 (antisense SSIII, kanamycin selection)

A fragment of the potato SSIII gene was isolated as a 1.14kb *Pst*I/*Eco*RV fragment from a partial cDNA clone for SSIII (Marshall et al, 1996 Plant Cell 8, 1121-1135; Accession number X95759). The SSIII fragment was sub-cloned in an antisense orientation between the cauliflower mosaic virus double 35S promoter and cauliflower mosaic virus terminator (*Pst*I/*Sma*I sites) in pJIT60 (Guerineau and Mullineaux, 1993) producing plasmid pRAT3. The *Xho*I-partial *Sst*I fragment from pRAT3, encompassing the promoter, antisense cDNA and terminator was ligated between the *Sal*I/*Sst*I sites of the plant transformation vector pBIN19 (Bevan, 1984), resulting in plasmid pRAT4 (Marshall *et al,* 1996 cited above).

### 2.1.3 pSJ119 (antisense SSIII, hygromycin selection)

A further vector (pSJ119) was constructed by cloning the 1.1 kb *Eco*RI fragment of pRAT3 containing the SSIII cDNA into the *Eco*RI site of pBSKSIIP (Stratagene) to create construct pSJ110 and then by inserting the 1.1 kb SSIII cDNA from pSJ110 (as an *Xba* I*-Sal* I fragment) in an antisense orientation under the control of the granule bound starch synthase (GBSS) promoter into the plant transformation vector pSJ39 which had been cut with the same enzymes. pSJ39 is a modified GPTV-HYG vector (Becker *et al* 1992 cited above) containing a 0.8 kb GBSS promoter and was constructed as follows: first the *Bam*HI site between the hygromycin selectable marker and the gene 7 polyadenylation signal in pGPTV-HYG was destroyed by cutting and filling in with klenow polymerase to create pSJ35. The *Hin*dIII*-Eco*RI fragment of this vector (containing the GUS gene & nos polyadenylation signal) was replaced with the *Hin*dIII*-Eco*RI fragment (containing the GBSS promoter-GUS-nos poly(A) cassette) from plasmid pGB121 (a gift from R. Visser, Wageningen) to create plasmid pSJ39. Plasmid pGB121 was constructed by inserting the 0.8 kb GBSS promoter from genomic clone LGBSSwt-6 (Visser et al 1989 Plant Science 64, 185-192) as an *Hin*dIII- *Nsi*I (klenow filled in) fragment into the *Hin*dIII- *Pst*I (T4 DNA polymerase blunted) sites of plasmid pBI121 (Jefferson et al 1987 EMBO J. **6**, 3901-3907).

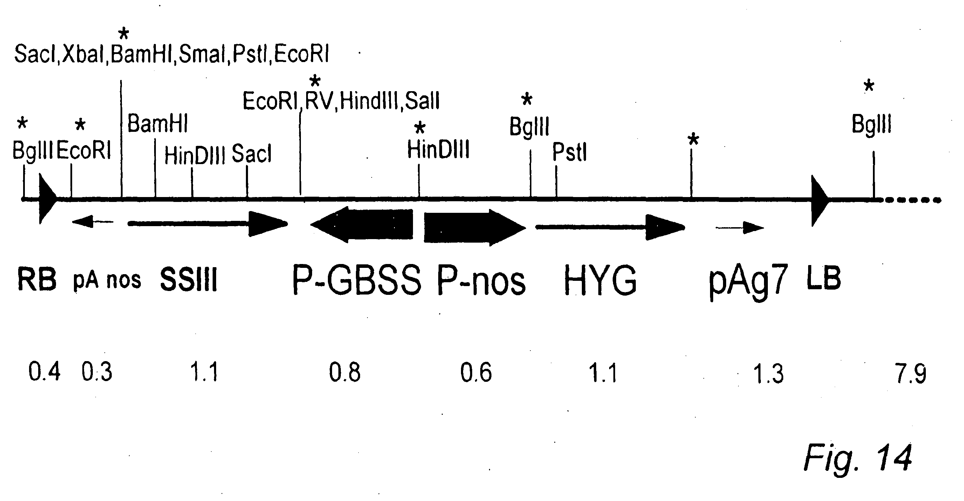

The plasmid pSJ 119 is illustrated schematically in Figure 14. Labelling follows the scheme adopted in Figure 1. Comparison of Figures 1 and 14 shows the similarity between plasmids pSJ152 and pSJ119.

### 2.2 Transformation of potato with pPOT3 (antisense GBSSI).

Binary plasmid pPOT3 was introduced into *Agrobacterium tumefasciens* LBA4404 by the freeze-thaw method of An et al (1988 Binary vectors. In Plant Molecular Biology Manual A3, Gelvin SB. Schilperoot RA, eds (Dordrecht, The Netherlands, Kluwer Academic Publishers) pp 1-19). Preparation of inoculum of Agrobacterium cells carrying pPOT3, inoculation of tuber discs of *Solanum tuberosum* cv Desiree, regeneration of shoots and rooting of shoots were as described in Edwards et al (1995 Plant J. 8, 283-294). *Agrobacterium* cells were grown in Luria broth containing rifampicin (50mg 1⁻¹). The growth medium for the tuber discs was Murashige and Skoog (MS) solution (Murashige and Skoog, 1962 cited above) containing 8g 1⁻¹ agar, zeatin riboside (5mg 1⁻¹) and indolacetic acid (0.1mg 1⁻¹), referred to as MS medium. The discs were co-cultivated for 2 days on tobacco feeder cell layers then transferred to MS medium containing cefotaxime (50g 1⁻¹) to select against *Agrobacterium.* After 5 days the discs were transferred to plates of MS medium containing cefotaxime (50g 1⁻¹) and kanamycin (100mg 1⁻¹) to select for growth of transformed plant cells.

Shoots were excised from the tuber discs when they had reached a height of at least 1cm and were rooted on 8g 1⁻¹ agar containing hormone-free MS and kanamycin (100mg 1⁻¹). Rooted shoots were sub-cultured twice before being transferred to a propagator and then to a greenhouse. When growth allowed sufficient tissue to be sampled, DNA was extracted from a few mgs of leaf and stem tissue of the culture using the method published by Edwards *et al* (1991 cited previously) and transformation was confirmed by PCR using gene specific primers. Material was left in culture to allow individual shoots to be micropropagated. Positive shoots were micropropagated to be stored *in vitro* and also to provide planting material. One line designated 4.3 was selected for further use as described below.

### 2.3 Transformation of wildtype potato with pRAT4 and waxy potato line 4.3 with pSJ119 (antisense SSIII).

Transformed potato plants cv Desiree were produced by co-cultivation of leaf fragments, produced from stock cultures with Agrobacterium tumefaciens LBA 4404 containing plant transformation vector plasmids. Stocks of plantlets were maintained by regular transfer of single node explants using Murashige and Skoog media (1962 Physiol. Plant 15. 473) solidified with 0.8% agar with the sucrose concentration reduced from 3% to 1%. All cultures were grown at 22°C with illumination from fluorescent tubes at 40µE/sq.m. for 16 hours except those to produce microtubers which were kept in darkness. The protocol for leaf explants was modified from that published by Rocha-Sosa et al (1989, EMBO J. 8. 23).

Explants were initially precultured on the appropriate multiplication medium for two days before co-cultivation. Co-cultivation was for 10 minutes in an overnight culture of the Agrobacterium before explants were blotted on filter paper to remove excess bacteria. After blotting explants were transferred to filter paper on feeder layers and incubated in darkness at 22°C for two days. The feeder layers consisted of a 9cm petri dish containing the appropriate multiplication media covered by 2ml of stationary phase tobacco suspension culture cells which were in turn covered by two layers filter paper.

After two days the explants were removed and washed in Murashige and Skoog medium without sucrose or agar containing 500µg/ml cefotaxime, blotted on filter paper and transferred to multiplication medium containing 500µg/ml cefotaxime. After a further five days the explants were further transferred to the same medium containing selective agent (kanamycin or hygromycin).

Tissue culture and selection using leaf explants was performed as follows. Expanded leaves were taken from stock cultures and cut into fragments comprising a half or a third of the leaf. The multiplication medium used contained the salts and vitamins of Murashige and Skoog medium plus 1.6% glucose, 0.02 mg/l naphthyl acetic acid, 2mg/l zeatin riboside and 0.8% agar. Kanamycin was added at 100mg/l when NPTII was used and hygromycin at 5mg/l when HPTII was used. Cultures were transferred every two weeks to the same medium. After four transfers the cultures which were vitrified or showed poor shoot development were transferred to liquid Murashige and Skoog medium containing 1.6% sucrose only.

Transformation was confirmed as follows. Shoots which developed after the multiplication/organogenesis phase were transferred individually to hormone-free Murashige and Skoog medium containing 1% sucrose and 100 mg/l kanamycin or 15mg/l hygromycin depending on the selection which was used. Only one shoot was taken from each explant unless the origin of separate shoots were well separated on the explant. Shoots which rooted on selection within two weeks were transfered to the same medium without selection and given an identifying number. Shoots were screened by PCR as described above. One SSIII antisense line designated 419-1 was selected for further study as were several GBSSI/SSIII antisense lines as described below.

### 2.4 Growth of Transformed Lines

Five plants from each line, when 50-100mm high, were transferred to compost made up of 50% horticultural sand and 50% Levington F2 peat-based compost in 20mm square modular pots and maintained at 20°C (day) and 15°C (night) in a growth room illuminated for 16 hours with high pressure sodium lamps at an illumination of 400µE/sq m. After watering they were covered within a small propagator and shaded from illumination. After 7-10 days when new growth was apparent the shading was removed and the plants grown on. With an intermediate potting to 70mm diameter pots finally the five plants were grown to maturity together in a 250mm diameter pot in Levington F2 compost under glass. Sixteen to eighteen weeks after transfer from culture, when the foliage began to die down, all the tubers were harvested. Representative samples were stored for analysis and possible regrowth and 150-200g fresh weight has taken for starch extraction.

### 2.5 Starch extraction and analysis

Washed tubers were diced and homogenised to a juice by passage through a Braun MP 75 centrifugal juicer. The juice was diluted with tap water to a volume of approximately 31 in a 51 conical flask. After 30 minutes the unsedimented supernatant liquid was discarded and the remaining solids washed again with a further 31 of water. After a further 30 minutes the liquid was decanted and discarded. The slurry of solids was poured through a 500µm metal mesh filter which retained the bulk of the cell debris. The crude starch which passed through the filter was washed in the same manner until no protein foam remained and only white starch settled on the base of the flask below a clear supernatant liquid. The starch was harvested on filter paper using a Buchner funnel and washed on the funnel with two 500ml volumes of distilled water. Finally the starch was washed with 500ml of acetone and dried in a fume hood for approximately 30 minutes.

RVA analysis was performed as described in Example 1.5 above. Differential scanning calorimetry with.a Perkin Elmer DSC 7 instrument was essentially as described in WO 95/26407, except that larger samples (10mgs) and larger pans were used, as explained in example 1.5 above.

### 2.6 Enzyme assay

### 2.6.1 Plant Material and Preparation of Plant Extracts

Tuber material was harvested from mature plants. After harvest the plant material was immediately frozen in liquid nitrogen and stored at - 70°C. Tubers were ground up in a coffee grinder which was precooled with dry ice. Extracts were made using four ml of extraction buffer per gram fresh weight tuber. The extraction buffer contained 100 mM Tris pH 7.5, 5% (v/v) glycerol, 2.5% (w/v) polyvinylpolypyrollidone (PVPP), 0.1 %(w/v) sodium metabisulphite, 2,5 mM DTT, 5 mM EDTA, 2mM Perfabloc, and 1mM benzamidine. Immediately after the addition of buffer, the samples were homogenised using a polytron (TM) homogenizer and clarified by centrifugation (15,000 rpm, 10 min, 4°C). The supernatant was used for assaying the soluble fraction of starch synthase activity as below.

### 2.6.2 Determination of Starch Synthase activities

The assay was based on the principle of ¹⁴C-labelled ADP-glucose incorporation into a growing glucan chain by the action of starch synthase. The assay mixture contained 100 mM Bicine pH 8.5, 25 mM potassium acetate, 5 mM EDTA, 2 mM DTT. 5 mg ml⁻¹ amylopectin, 0.5 M citrate (optional), 1 mM ADP-glucose (2.31 GBq mole⁻¹ = 2.31x10⁻⁴ MBq per assay) and 10-20 µl of extract in a final assay volume of 100 µl. Each extract was assayed in duplicate to check the reproducibility of the sample handling. Controls contained boiled extracts. After an incubation time of 15 to 20 minutes at 25 °C the reaction was stopped by boiling for 2 minutes. After a short spin (to remove radioactive reaction mixture from the lid before opening), 1 ml of 75% methanol / 1% KCl was added to precipitate the synthesised glucans. After 3 minutes the precipitates were centrifuged (for 5 min at 6,500 rpm). The supernatant was discarded and the pellet washed once with 1 ml 75% methanol / 1% KCl. The final pellet was dissolved in 100 µl DMSO. To speed up this process, a few grains of sand were added and the tubes put on a shaker for 30 to 60 min. After all pellets were dissolved, 1.25 ml of ReadySafe™ scintillation cocktail was added. The tubes were placed in larger scintillation vials and counted for one minute. Units of enzyme activity were expressed in nmole glucose min⁻¹ and calculated as follows:
U/gmFresh Weight = cpm/2200 x 16 x 1/t x 1000/v x f
U = nmole glucose min⁻¹, cpm = counts per minute, t = time in minutes, v = extract volume in the assay in µl, f = extraction factor (ml of extraction buffer used to extract 1 g of sample)

### 2.6.3 Starch synthase zymogram

Zymograms were used to determine the presence of different isoforms of starch synthases. This was done by separating the enzymes (crude extract) by a non-denaturing gel electrophoresis followed by an incubation in enzyme substrates (primer, ADP-glucose. Enzyme activity was visualised by staining the gel with an iodine/iodide solution (see below), starch synthases being identified by the formation of blue bands. Native enzyme extracts are separated on a native polyacrylamide gel (Laemmli gel without SDS) using a BioRad Mini Protean (TM) II gel chamber. The gel consists of a 7.5% separation gel (7.5% acrylamide, 0.375M Tris-HCl pH 8.8, 10% (w/v) glycerol; 0.1% ammonium persulfate, 0.05% TEMED) and a 4% stacking gel (4% acrylamide, 0.125 M Tris-HCl pH 6.8, 10% (w/v) glycerol; 0.05% ammonium persulfate, 0.1% TEMED). Crude extracts (see above) are spiked with some bromophenol blue and about 50-80 µg (40 µl) is loaded onto the gel. The electrophoresis is carried out at 4°C at a constant voltage of 100 V using a 1x electrophoresis buffer without SDS (0.3028% (w/v) Tris, 1.44 %(w/v) glycine). Electrophoresis is stopped when the bromophenol blue reaches the bottom of the gel (about 3.5h). The gel is then incubated over night at room temperature in 30 ml of incubation buffer (50 mM Bicine pH 8.5, 0.5 M Na-citrate, 25 mM K-acetate, 2 mM DTT, 2 mM EDTA, 0.1 % amylopectin, 1 mM ADP-glucose) on a slowly moving shaker. After incubation the gel is rinsed in water and stained with iodine/iodide solution (0.1% KI, 0.01 % I₂).

### 2.7 Results

Several antisense GBSSI lines were developed and one line, designated as line 4.3, was selected for further use since it had very low GBSS activity, approximately 10% that of wildtype, had a severe reduction in the waxy granule bound 60 kDa protein, and the starch from this line stained red with iodine indicating low levels of amylose. This line was retransformed with the SSIII antisense construct pSJ119. Plants were identified with a four digit number; 0801-0809 were regrowths of the original GBSSI line 4.3 whereas 0810 onwards were doubly transformed lines (GBSSI/SSIII antisense). One SSIII antisense line designated 419-1 which had very low levels of SSIII (as assayed by zymogram analysis, data not shown) was also selected as a control. These plants, along with wild type control lines designated 1603 and 0302, were grown to maturity in the greenhouse, tubers harvested and starch was extracted and analysed. Sixty-one doubly transformed waxy (4.3) plants were selected, based on their resistance to hygromycin selection and PCR screening for the presence of the SSIII antisense T-DNA. Zymogram analysis of the soluble fraction of tuber extracts showed that ten of these lines had very low levels of SSIII (data omitted for brevity). The physical properties of starch from these lines was analysed by RVA and DSC and a summary of the analysis is shown in Table 3. All of these starches had zero amylose content as assayed by the potentiometric iodine based method as described by Shi et al (1998 J. Cereal Sci. 27, 289-299) whereas the control starch (0302) had an amylose content of 25.59%.

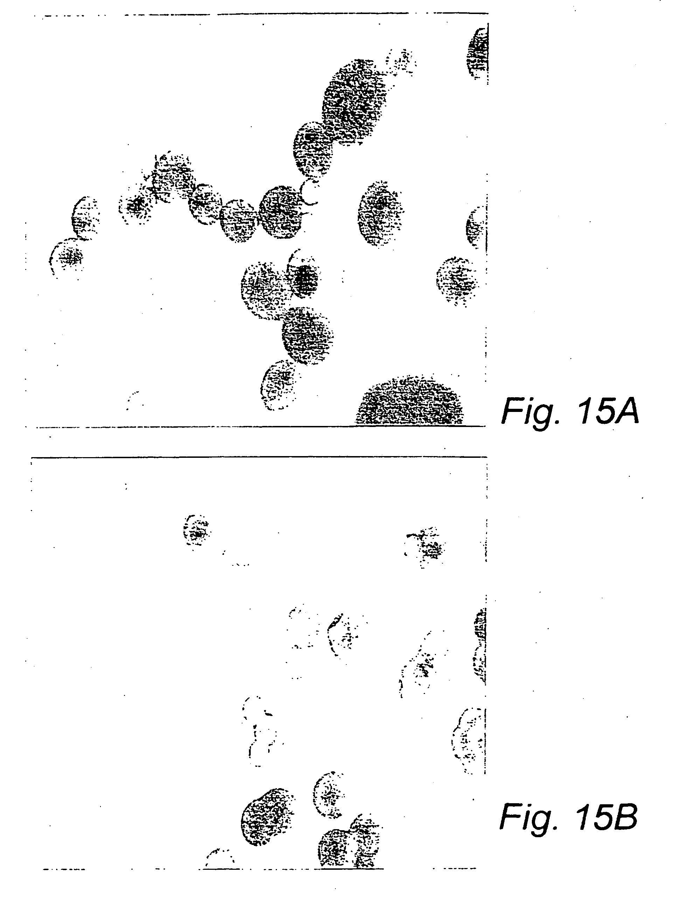

When the starch granules were stained with iodine, all the waxy starches appeared predominately red with a small central blue core in some of the granules. again indicating very low amylose levels (Figures 15A, 15B).

Figures 15A and B are micrographs (taken at the same level of magnification of iodine-stained starch granules from the GBSSI antisense line 0805 (Fig. 15A) and the GBSSI/SSIII line 08103 (Fig. 15B).

Reduction of SSIII expression in the waxy lines had a large effect on granule morphology. Whereas the waxy control 0805 starch granules were elliptical in shape and fairly uniform in size, the majority of the starch granules from the waxy plants that had very low levels of SSIII were much more spherical and there was larger variation in granule size with many more small granules which often had angular faces. These smaller starch granules were often found in association with other granules either in small clumps or rows. The angular faces appeared to form at the junction of the associated granules. It should be noted that the granule morphology of waxy lines with low levels of SSIII is very different from starches which only have reduced levels of SSIII which, as reported previously, have deeply cracked granules as well as many clusters of small granules (Marshall et al 1996).

Viscometric analysis showed that the waxy control lines all showed the typical sharp peak with a slightly increased viscosity onset temperature and lower setback (final viscosity) compared to starch from the wildtype control. The onset of gelatinisation and endotherm peak of the waxy starch as measured by DSC was also significantly higher than wild type controls (Table 3). Amylose-free potato starch has previously been reported to display similar properties (Visser and Suurs 1997 Starch/Starke 49, 443-448).

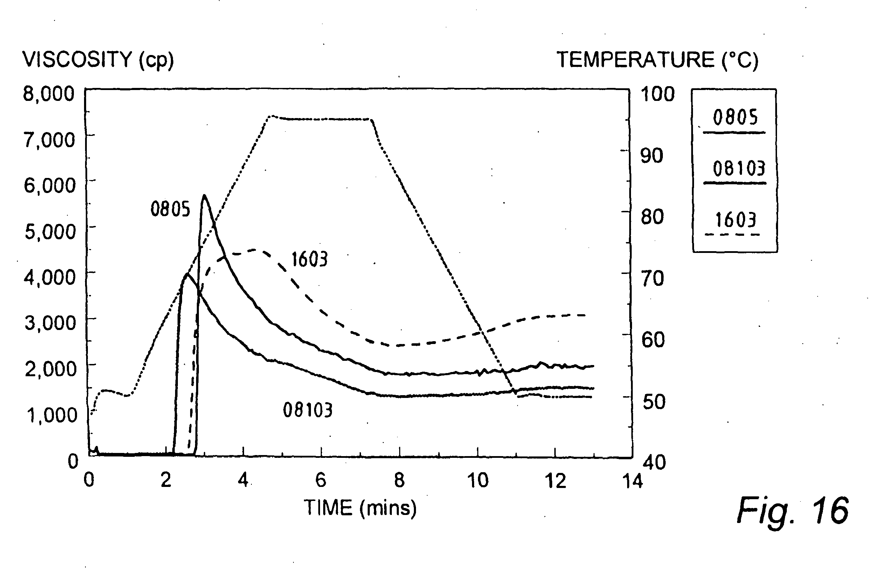

Starch from the waxy plants that had very low levels of SSIII however showed significantly different properties; an example of an RVA profile of starch from line 08103 is shown in Fig. 16. Figure 16 is a graph showing the results of viscometric analysis of starch from three different plants. The Figure takes the form of a graph of viscosity in cP (left hand axis) or temperature in °C (right hand axis) against time (in minutes). The temperature profile is shown by the dotted line, and the viscoamylographs for starch from plants 0805, 08103 and 1603 are represented by a thin solid line, a thick solid line and a dashed line respectively. The viscosity onset temperature of starch from line 08103 was at least 7°C lower than the waxy starch although the sharp peak characteristic of waxy type starch was still evident, but the peak viscosity was lower, as was the final viscosity.

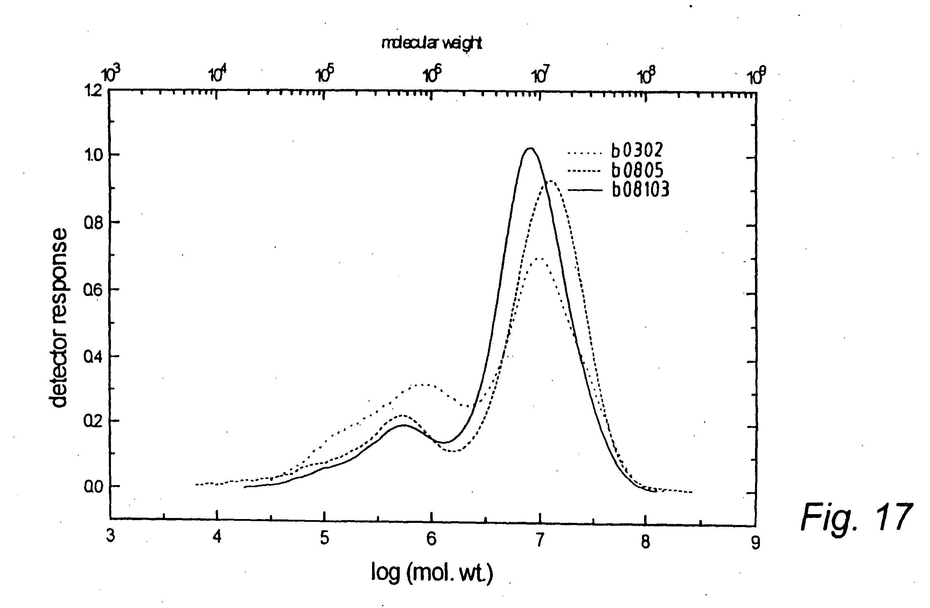

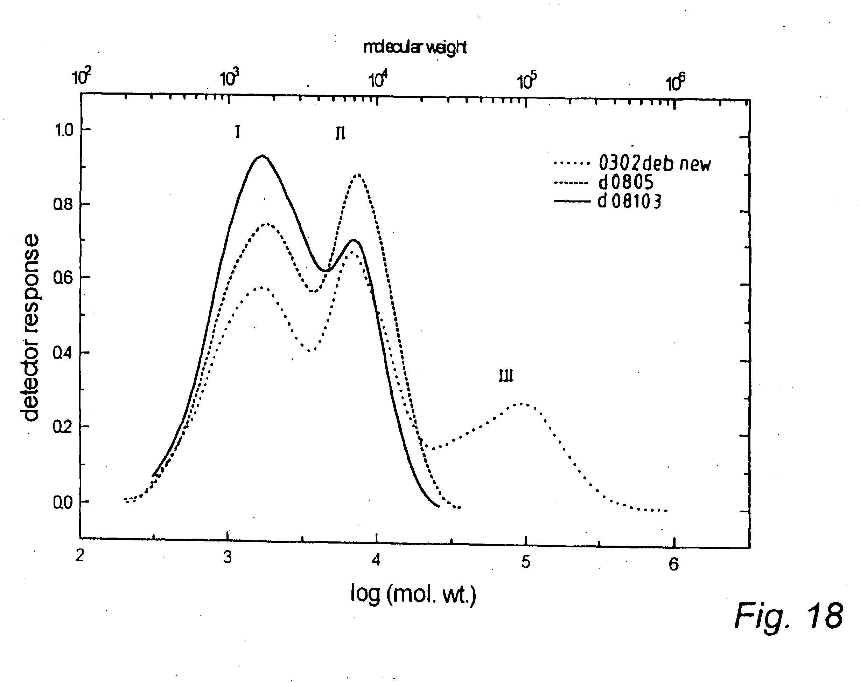

The onset of gelatinisation and endotherm peak of starch from 08103 as measured by DSC was also decreased by about 7°C compared to the waxy control (0805, Table 3). The molecular structure of the starches was analysed in more detail by gel permeation chromatography either before or after debranching with isoamylase. The results of such GPC analysis are shown in Figures 17 and 18 respectively.

Figures 17 and 18 are graphs of detector response (arbitary units) against molecular weight, and show the results of GPC analysis of starch from plants 0302 (widely spaced dotted line); 0805 (closely-spaced dotted line); and 08103 (solid line).

Starch from the waxy/SSIII lines 08103, 08104, 08131, 08133 and 08146 was analysed and all showed very similar profiles so only the results from line 08103 are shown for clarity. Wild type starch (0302) was fractionated into an amylopectin fraction with a peak at around mol. wt. of 10⁷ and an amylose containing fraction with a peak at around 10⁶. Starch from 08103 appeared very similar to the waxy control (0805) as both showed a large amylopectin peak and a much smaller fraction at around 10⁵-10⁶ (Figure 17).

After debranching neither of these starches showed any significant quantity of long linear chains (fraction III Fig. 18) indicating a lack of amylose in these starches. The shorter chains (fractions I and II) are derived from the branches of amylopectin. Integration of the area under the peaks of fraction I and II shows that in both the control and waxy 0805 starches the shorter branches (fraction I) are slightly more abundant than the longer branches in fraction II (54 % and 46 % respectively). However in the GBSSI/SSIII line 08103 the proportion of short branches in fraction I increased significantly such that this fraction contained 69 % of the total vs 31 % in fraction II.

**Table 3. Summary of RVA and DSC analysis of starches**

| Code | RVA DATA | | | | | DSC DATA | | | | | |
|---|---|---|---|---|---|---|---|---|---|---|---|
| | Onset | Peak | Peak | Pasting | Setback | Peak | s.e. | delta H | s.e. | Onset | s.e. |
| | (°C) | (°C) | (cP) | (cP) | (cP) | (°C) | | (J/g) | | (°C) | |
| 1603 | 67.8 | 89.6 | 4488 | 2483 | 3091 | 68.18 | 0.22 | 22.35 | 0.04 | 63.97 | 0.12 |
| 0302 | | | | | | 68.36 | 0.05 | 22.17 0.03 | | 64.30 | 0.06 |
| | | | | | | | | | | | |
| 0805 | 71.1 | 75.2 | 5688 | 1909 | 1986 | 72.30 | | 20.13 | | 68.96 | |
| | | | | | | | | | | | |
| 0806 | 71.0 | 75.9 | 5320 | 1677 | 1977 | 72.60 | | 21.20 | | 69.16 | |
| 0880 | 66.2 | 72.6 | 3706 | 1811 | 1884 | 67.86 | 0.05 | 18.89 | 0.09 | 64.23 | 0.11 |
| 0891 | 67.0 | 73.4 | 4488 | 1841 | 2111 | 68.50 | 0.02 | 19.20 | 0.02 | 64.06 | 0.13 |
| 0892 | 66.2 | 71.1 | 4952 | 1893 | 2106 | 67.63 | 0.13 | 19.53 | 0.21 | 64.21 | 0.03 |
| 0897 | 65.3 | 69.5 | 4845 | 1411 | 1589 | 66.64 | 0.02 | 19.94 | 0.69 | 62.93 | 0.16 |
| 08103 | 63.8 | 69.4 | 3963 | 1384 | 1503 | 65.09 | 0.33 | 18.30 | 0.01 | 61.14 | 0.31 |
| 08104 | 64.6 | 69.5 | 4351 | 1525 | 1613 | 65.31 | 0.19 | 20.29 | 0.47 | 62.13 | 0.09 |
| 08124 | 64.5 | 72.6 | 4449 | 1762 | 2046 | 66.30 | 0.01 | 19.43 | 0.22 | 62.36 | 0.05 |
| 08131 | 65.4 | 69.5 | 4731 | 1352 | 1548 | 66.41 | 0.01 | 19.05 | 0.30 | 63.14 | 0.04 |
| 08133 | 64.5 | 70.9 | 4098 | 1733 | 1983 | 65.94 | 0.06 | 19.46 | 0.07 | 62.40 | 0.07 |
| 08146 | 66.2 | 81.6 | 4152 | 2356 | 2398 | 69.89 | 0.39 | 19.79 | 0.01 | 62.96 | 0.26 |

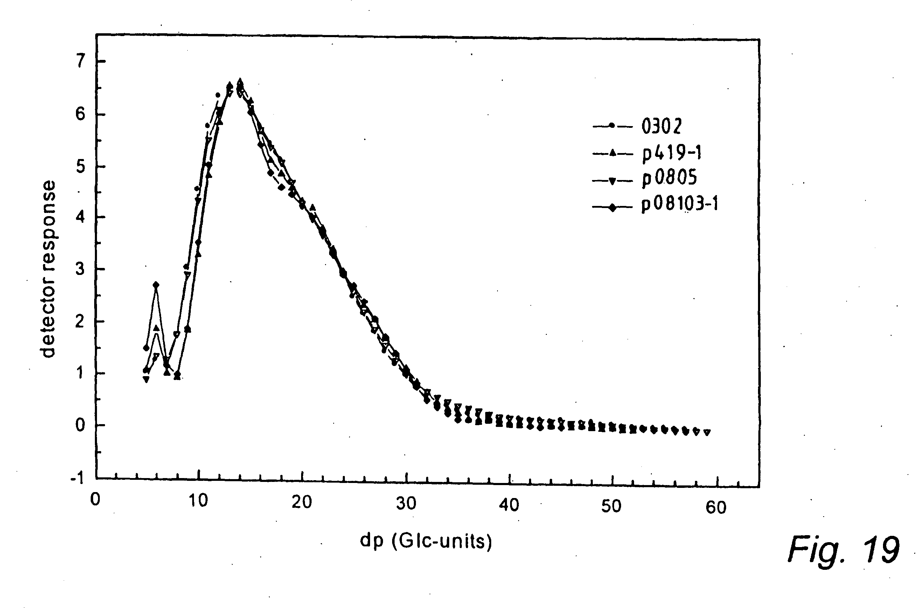

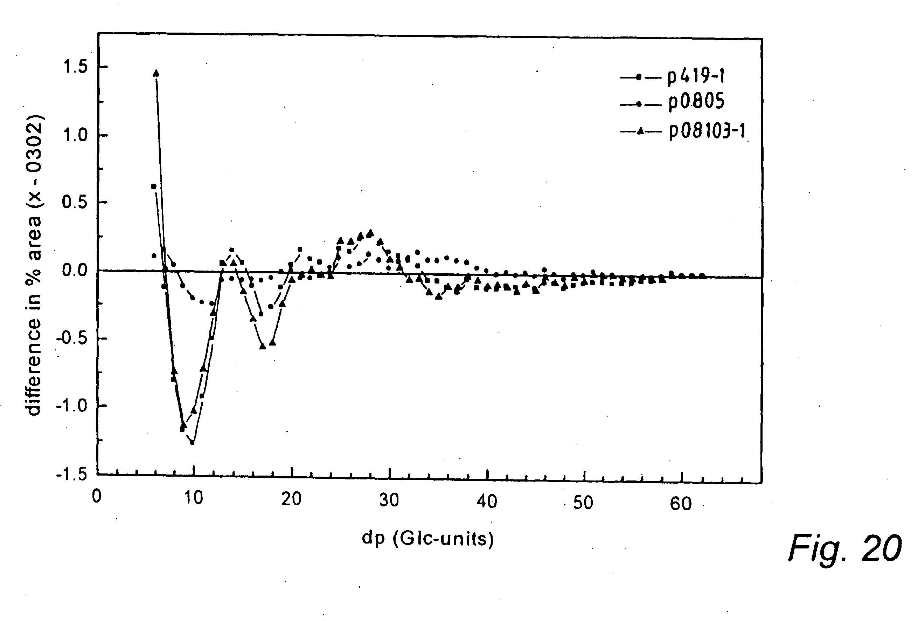

The chain length distribution of these starches was further examined by HPAEC using the dionex system, as described in Example 1, and the results are shown in Figure 19, which is a graph of detector response (arbitary units) against degree of polymerisation (dp). The control starch 0302 (round symbols) has a minimum at around dp 7 and a maximum at dp13-14. The chain length distribution of the waxy starch 0805 (downward-pointing triangles) is not significantly different from the control. However, starch from line 08103 (lozenge symbols) shows an altered distribution, with more chains of dp 6, and less of dp 8-12 and 16-19 and is very similar to starch from line 419-1 (upward-pointing triangles) in which only SSIII is reduced. These changes are more easily seen when the results are shown as a difference plot (Figure 20). This Figure was produced by subtracting the % area of each peak in the control (0302) from the selected sample data, and takes the form of a plot of difference in % area against dp. Results for 419-1, 0805 and 08103 are shown by squares, circles and triangles, respectively.

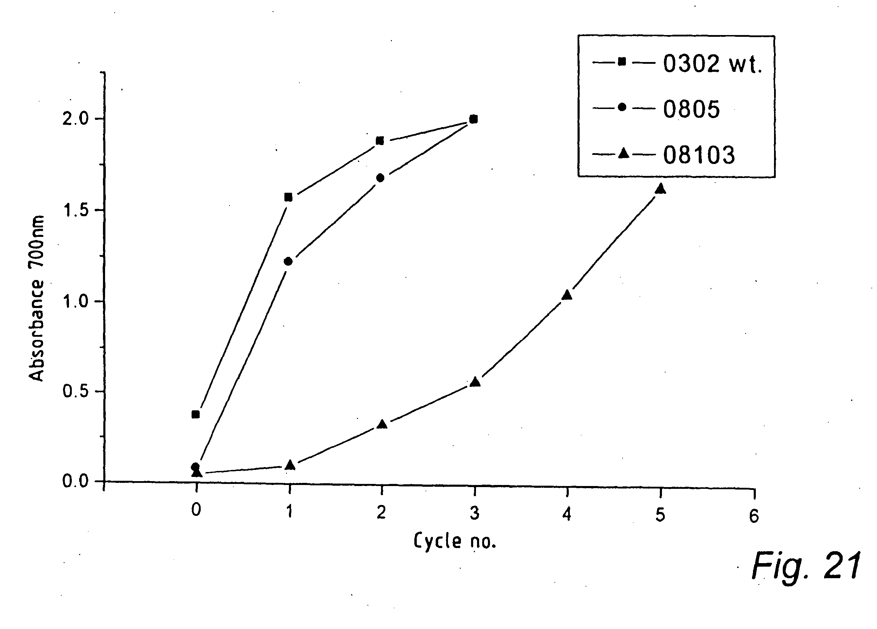

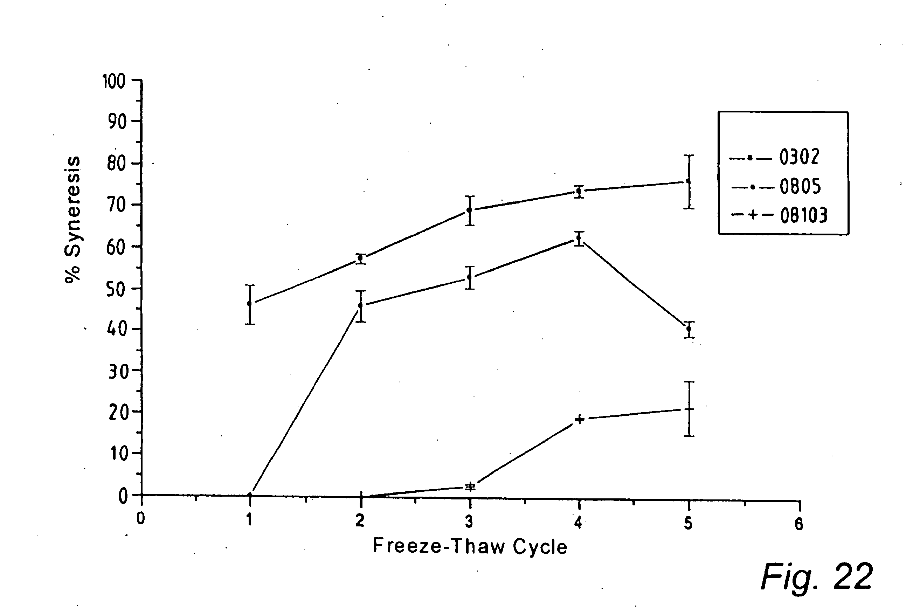

The freeze-thaw stability of the GBSSI/SSIII starch was assessed as described in Example 1.2.5, except that much shorter freeze-thaw cycles were employed. Samples were frozen for 1 hour (at -70°C) followed by a 10 minute thaw. Figure 21 shows the freeze thaw stability based on absorbance, whereas Figure 22 shows the syneresis data.

Figure 21 is a graph of absorbance (at 700nm) against number of freeze-thaw cycles. Figure 22 is a graph of % syneresis against number of freeze-thaw cycles. The plots for starch from 0302 is shown in both Figures by square symbols, and those for 0805 by circles. In Figure 21, data for 08103 is shown by triangles, whilst cross symbols are used in Figure 22.

The control starches show very similar freeze-thaw properties using this quick freeze-thaw cycle as compared to the long freeze-thaw cycle (compare Figs. 11 & 12 with Figs. 21 & 22). The GBSSI/SIII starch from line 08103 shows some freeze thaw stability in Figure 21 as there is very little increase in absorbance after one freeze-thaw cycle but after this the absorbance increases significantly and after five cycles the absorbance of this starch is approximately the same as the controls. However, as measured by syneresis (Figure 22), the GBSSI/SSIII starch 08103 is completely freeze-thaw stable for at least two cycles.

After three cycles a very small amount of syneresis is observed and this increases significantly in the fourth and fifth cycles although the increase is much smaller than seen in the control starches (wild type 0302 and waxy 0805).

## Claims

1. A potato plant cell comprising introduced nucleic acid sequences, which sequences specifically inhibit the expression of granule bound starch synthase I (GBSSI) and at least one further starch synthase enzyme involved in starch synthesis in the potato plant cell.

2. A potato plant cell according to claim 1, wherein the said at least one further starch synthase enzyme is specifically inhibited by the introduced nucleic acid sequences comprising starch synthase II and/or starch synthase III.

3. A potato plant cell according to claim 1 comprising introduced nucleic acid sequences, which sequences specifically inhibit the expression of three or more enzymes involved in starch synthesis in the cell.

4. A potato plant comprising introduced nucleic acid sequences, wherein the introduced nucleic acid sequences specifically inhibit the expression of GBSSI and at least one further starch synthase enzyme involved in starch synthesis in the potato plant.

5. A potato plant according to claim 4, in which the introduced nucleic acid sequences specifically inhibit GBSSI, and one or both of SSII and SSIII.

6. The progeny of a potato plant comprising introduced nucleic acid sequences, which progeny retains the introduced sequences, wherein the introduced nucleic acid sequences specifically inhibit the expression of GBSSI and at least one further starch synthase enzyme involved in starch synthesis in the potato plant.

7. The progeny of a potato plant according to claim 6, in which the introduced nucleic acid sequences specifically inhibit GBSSI, and one or both of SSII and SSIII.

8. A potato plant according to claim 5, produced from a potato plant cell according to any one of claims 1-3.

9. A potato plant according to claim 8, which gives rise to starch which has an apparent amylose content of less than 8%, as determined by the method of Morrison & Laignelet (1983 Cereal Science 1, 9-20), and a viscosity onset temperature of less than 67°C as determined by viscometric analysis of a 7.4% (w/v) aqueous suspension of the starch using a Rapid Visco Amylograph, Newport Scientific Series 4 instrument operating on the standard 1 heating and stirring protocol.

10. A potato plant according to claim 8 which gives rise to starch as defined in claim 9 and which, when extracted, exhibits freeze-thaw stability such that a 1 %w/v aqueous suspension of the starch has an absorbance at 700nm wavelength of less than 1.2 units following 4 freeze/thaw cycles of freezing at -70°C overnight and thawing at room temperature for at least 2 hours.

11. A potato plant according to claim 8 which gives rise to starch as defined in claim 9, and wherein 1%w/v aqueous suspension of the starch has an absorbance at 700nm wavelength of less than 1.0 units following 4 freeze/thaw cycles.

12. A potato plant according to claim 8 which gives rise to starch as defined in claim 9 which, when extracted, exhibits freeze-thaw stability such that a 1 %w/v aqueous suspension of the starch has an absorbance at 700nm wavelength of less than 0.9 units following 3 freeze/thaw cycles of freezing at -70°C overnight and thawing at room temperature for at least two hours.

13. A potato plant according to claim 8 which gives rise to starch as defined in claim 12, wherein a 1% w/v aqueous suspension of the starch has an absorbance at 700nm wavelength of less than 0.7 units following 3 freeze/thaw cycles.

14. A potato plant according to claim 8 which gives rise to starch as defined in claim 9 which, when extracted, exhibits freeze-thaw stability such that a 1 % w/v aqueous suspension of the starch has an absorbance at 700nm wavelength of less than 0.7 units following 2 freeze/thaw cycles of freezing at -70°C overnight and thawing at room temperature for at least two hours.

15. A potato plant according to claim 8 which gives rise to starch as defined in claim 14, wherein a 1% w/v aqueous suspension of the starch has an absorbance at 700nm wavelength of less than 0.5 units following 2 freeze/thaw cycles.

16. A potato plant according to claim 8 which gives rise to starch as defined in claim 9 which, when extracted, exhibits freeze-thaw stability such that a 1 %w/v aqueous suspension of the starch has an absorbance at 700nm wavelength of less than 0.5 units following 1 freeze/thaw cycle of freezing at -70°C overnight and thawing at room temperature for at least 2 hours.

17. A potato plant according to claim 8 which gives rise to starch as defined in claim 16, wherein a 1% w/v aqueous suspension of the starch has an absorbance at 700nm wavelength of less than 0.3 units following 1 freeze/thaw cycle.

18. A potato plant according to claim 8 which gives rise to starch as defined in claim 9, which, when extracted, exhibits freeze-thaw stability, such that a 5% w/v aqueous paste of the starch exhibits less than 40% syneresis following 4 freeze/thaw cycles of freezing at -70°C overnight and thawing at 22°C for 60 minutes, and then spinning at 8,000g for 10 minutes at 18°C.

19. A potato plant according to claim 8 which gives rise to starch as defined in claim 18, which exhibits less than 30% syneresis following 4 freeze/thaw cycles.

20. A potato plant according to claim 8 which gives rise to starch as defined in claim 18, which exhibits less than 20% syneresis following 4 freeze/thaw cycles.

21. A potato plant according to claim 8 which gives rise to starch as defined in claim 18, which exhibits less than 10% syneresis following 4 freeze/thaw cycles.

22. A potato plant according to claim 8 which gives rise to starch as defined in claim 9 which, when extracted, exhibits freeze-thaw stability, such that a 5% w/v aqueous paste of the starch exhibits less than 30% syneresis following 3 freeze/thaw cycles of freezing at -70°C overnight and thawing at 22°C for 60 minutes, and then spinning at 8,000g for 10 minutes at 18°C.

23. A potato plant according to claim 8 which gives rise to starch as defined in claim 22, which exhibits less than 20% syneresis following 3 freeze/thaw cycles.

24. A potato plant according to claim 8 which gives rise to starch as defined in claim 22, which exhibits less than 10% syneresis following 3 freeze/thaw cycles.

25. A potato plant according to claim 8 which gives rise to starch as defined in claim 9 which, when extracted, exhibits freeze-thaw stability, such that a 5% w/v aqueous paste of the starch exhibits less than 30% syneresis following 2 freeze/thaw cycles of freezing at -70°C overnight and thawing at 22°C for 60 minutes, and then spinning at 8,000g for 10 minutes at 18°C.

26. A potato plant according to claim 8 which gives rise to starch as defined in claim 25, which exhibits less than 20% syneresis following 2 freeze/thaw cycles.

27. A potato plant according to claim 8 which gives rise to starch as defined in claim 25, which exhibits less than 10% syneresis following 2 freeze/thaw cycles.

28. A potato plant according to claim 8 which gives rise to starch as defined in claim 9 which, when extracted, exhibits freeze-thaw stability, such that a 5% w/v aqueous paste of the starch exhibits less than 40% syneresis following 4 freeze/thaw cycles of freezing at -70°C for 1 hour and thawing at 22°C for 10 minutes, and then spinning at 8,000g for 10 minutes at 18°C.

29. A potato plant according to claim 8 which gives rise to starch as defined in claim 28, which exhibits less than 30% syneresis following 4 freeze/thaw cycles.

30. A potato plant according to claim 8 which gives rise to starch as defined in claim 28, which exhibits less than 20% syneresis following 4 freeze/thaw cycles.

31. A potato plant according to claim 8 which gives rise to starch as defined in claim 9 which, when extracted, exhibits freeze-thaw stability, such that a 5% w/v aqueous paste of the starch exhibits less than 40% syneresis following 3 freeze/thaw cycles of freezing at -70°C for 1 hour and thawing at 22°C for 10 minutes, and then spinning at 8,000g for 10 minutes at 18°C.

32. A potato plant according to claim 8 which gives rise to starch as defined in claim 31, which exhibits less than 30% syneresis following 3 freeze/thaw cycles.

33. A potato plant according to claim 8 which gives rise to starch as defined in claim 31, which exhibits less than 20% syneresis following 3 freeze/thaw cycles.

34. A potato plant according to claim 8 which gives rise to starch as defined in claim 31, which exhibits less than 10% syneresis following 3 freeze/thaw cycles.

35. A potato plant according to claim 8 which gives rise to starch as defined in claim 9 which, when extracted, exhibits freeze-thaw stability, such that a 5% w/v aqueous paste of the starch exhibits less than 30% syneresis following 2 freeze/thaw cycles of freezing at -70°C for 1 hour and thawing at 22°C for 10 minutes, and then spinning at 8,000g for 10 minutes at 18°C.

36. A potato plant according to claim 8 which gives rise to starch as defined in claim 29, which exhibits less than 20% syneresis following 2 freeze/thaw cycles.

37. A potato plant according to claim 8 which gives rise to starch as defined in claim 35, which exhibits less than 10% syneresis following 2 freeze/thaw cycles.

38. A potato plant according to claim 8 which gives rise to starch as defined in claim 9 which, when extracted, has a ratio of fraction I to fraction II short chain glucans of at least 60%.

39. A potato plant as defined in claim 8 which gives rise to starch as defined in claim 38, having a fraction I to fraction II ratio of at least 65 %.

40. A potato plant according to claim 8 which gives rise to starch as defined in claim 38, having a fraction I to fraction II ratio of at least 70%.

41. A potato plant according to claim 8 which gives rise to starch as defined in claim 9, having a viscosity onset temperature of less than 65°C.

42. A potato plant according to claim 8 which gives rise to starch as defined in claim 9, having a viscosity onset temperature of less than 55°C.

43. A potato plant according to claim 8 which gives rise to starch as defined in claim 9, having a viscosity onset temperature of less than 51°C.

44. A potato plant according to claim 8 which gives rise to starch as defined in claim 9 which, when analysed by differential scanning calorimetry using a Perkin Elmer DSC7 instrument a 10mg starch sample in aqueous mix of less than 25% starch w/v exhibits a gelatinisation onset temperature of less than 67°C.

45. A potato plant according to claim 8 which gives rise to starch as defined in claim 44, which exhibits a gelatinisation onset temperature of less than 66°C.

46. A potato plant according to claim 8 which gives rise to starch as defined in claim 44, which exhibits a gelatinisation onset temperature of less than 51°C.

47. A potato plant according to claim 8 which gives rise to starch as defined in claim 44, which exhibits a gelatinisation onset temperature of less than 50°C.

48. A potato plant according to claim 8 which gives rise to starch as defined in any one of claims 8-47, wherein the starch granules are free of cracks.

49. A method of altering the starch content of a potato plant, the method comprising the steps of: providing nucleic acid sequences; and introducing said sequences into the potato plant, wherein the sequences specifically inhibit the expression of GBSSI and at least one further enzyme involved in starch synthesis in the potato plant.

50. A method according to claim 49, wherein the sequences are introduced into a potato plant cell, and the plant cell is grown into a potato plantlet and subsequently into a potato plant.

51. A method according to claim 49 or 50, wherein the introduced sequences specifically inhibit the expression of GBSSI and one or both of SSII and SSIII.

52. A method according to any one of claims 49-51, wherein the introduced nucleic acid sequences are operably linked in the antisense orientation to a promoter active in the potato plant, so as to cause transcription of the sequences.

53. A method of making starch, the method comprising the steps of: altering the starch content of a potato plant by the method of any one of claims 49-51; and extracting the altered starch content from the potato plant.

54. A method according to claim 53, further comprising the step of modifying the extracted starch by physical, and/or enzymatic and/or chemical processing *in vitro.*

55. A potato tuber produced by a potato plant progeny according to claim 6 or 7, and comprising a potato plant cell according to claims 1-3.

56. A potato tuber produced by a potato plant according to any one of claims 9-48 and comprising a potato plant cell according to claims 1-3.

## Patentansprüche

1. Kartoffelpflanzenzelle umfassend eingeführte Nukleinsäuresequenzen, die spezifisch die Expression der stärkekorngebundenen Stärkesynthase I (GBSSI) sowie mindestens eines weiteren Stärkesynthaseenzyms, das an der Stärkesynthese in der Kartoffelpflanzenzelle beteiligt ist, hemmen.

2. Kartoffelpflanzenzelle nach Anspruch 1, wobei das mindestens eine weitere Stärkesynthaseenzym spezifisch durch die eingeführten Nukleinsäuresequenzen umfassend die Stärkesynthase II und/oder Stärkesynthase III gehemmt wird.

3. Kartoffelpflanzenzelle nach Anspruch 1, umfassend eingeführte Nukleinsäuresequenzen, die spezifisch die Expression von drei oder mehr Enzymen, die an der Stärkesynthese in der Zelle beteiligt sind, hemmen.

4. Kartoffelpflanze umfassend eingeführte Nukleinsäuresequenzen, wobei die eingeführten Nukleinsäuresequenzen spezifisch die Expression der GBSSI und mindestens eines weiteren Stärkesynthaseenzyms, das an der Stärkesynthese in der Kartoffelpflanze beteiligt ist, hemmen.

5. Kartoffelpflanze nach Anspruch 4, in der die eingeführten Nukleinsäuresequenzen spezifisch die GBSSI sowie die SSII und/oder SSIII hemmen.

6. Nachkommenschaft einer Kartoffelpflanze umfassend eingeführte Nukleinsäuresequenzen, die die eingeführten Sequenzen beibehält, wobei die eingeführten Nukleinsäuresequenzen spezifisch die Expression der GBSSI und mindestens eines weiteren Stärkesynthaseenzyms, das an der Stärkesynthese in der Kartoffelpflanze beteiligt ist, hemmen.

7. Nachkommenschaft einer Kartoffelpflanze nach Anspruch 6, in der die eingeführten Nukleinsäuresequenzen spezifisch die GBSSI sowie die SSII und/oder SSIII hemmen.

8. Kartoffelpflanze nach Anspruch 5, die aus einer Kartoffelpflanzenzelle nach einem der Ansprüche 1-3 erzeugt wurde.

9. Kartoffelpflanze nach Anspruch 8, die zu einer Stärke mit einem scheinbaren Amylosegehalt von weniger als 8% gemäß dem Verfahren von Morrison & Laignelet (1983 Cereal Science 1, 9-20) und einer Viskositäts-Onset-Temperatur von weniger als 67°C gemäß viskometrischer Analyse einer 7,4%igen (w/v) wäßrigen Suspension der Stärke mit dem Rapid Visco Amylographen, Newport Scientific Series 4, der mit dem Standard 1 Aufheiz- und Rührprotokoll betrieben wird, führt.

10. Kartoffelpflanze nach Anspruch 8, die zu einer Stärke gemäß Anspruch 9 führt und die bei Extraktion eine derartige Gefrier-Tau-Stabilität aufweist, daß eine 1%ige w/v wäßrige Suspension der Stärke nach 4 Gefrier-Tau-Zyklen von Gefrieren über Nacht bei -70°C und Auftauen bei Raumtemperatur über mindestens 2 Stunden eine Extinktion von weniger als 1,2 Einheiten bei einer Wellenlänge von 700 nm aufweist.

11. Kartoffelpflanze nach Anspruch 8, die zu einer Stärke gemäß Anspruch 9 führt und in der eine 1%ige w/v wäßrige Suspension der Stärke nach 4 Gefrier-Tau-Zyklen eine Extinktion von weniger als 1,0 Einheiten bei einer Wellenlänge von 700 nm aufweist.

12. Kartoffelpflanze nach Anspruch 8, die zu einer Stärke gemäß Anspruch 9 führt und die bei Extraktion eine derartige Gefrier-Tau-Stabilität aufweist, daß eine 1%ige w/v wäßrige Suspension der Stärke nach 3 Gefrier-Tau-Zyklen von Gefrieren über Nacht bei -70°C und Auftauen bei Raumtemperatur über mindestens zwei Stunden eine Extinktion von weniger als 0,9 Einheiten bei einer Wellenlänge von 700 nm aufweist.

13. Kartoffelpflanze nach Anspruch 8, die zu einer Stärke gemäß Anspruch 12 führt und in der eine 1%ige w/v wäßrige Suspension der Stärke nach 3 Gefrier-Tau-Zyklen eine Extinktion von weniger als 0,7 Einheiten bei einer Wellenlänge von 700 nm aufweist.

14. Kartoffelpflanze nach Anspruch 8, die zu einer Stärke gemäß Anspruch 9 führt und die bei Extraktion eine derartige Gefrier-Tau-Stabilität aufweist, daß eine 1%ige w/v wäßrige Suspension der Stärke nach 2 Gefrier-Tau-Zyklen von Gefrieren über Nacht bei -70°C und Auftauen bei Raumtemperatur über mindestens zwei Stunden eine Extinktion von weniger als 0,7 Einheiten bei einer Wellenlänge von 700 nm aufweist.

15. Kartoffelpflanze nach Anspruch 8, die zu einer Stärke gemäß Anspruch 14 führt und in der eine 1%ige w/v wäßrige Suspension der Stärke nach 2 Gefrier-Tau-Zyklen eine Extinktion von weniger als 0,5 Einheiten bei einer Wellenlänge von 700 nm aufweist.

16. Kartoffelpflanze nach Anspruch 8, die zu einer Stärke gemäß Anspruch 9 führt und die bei Extraktion eine derartige Gefrier-Tau-Stabilität aufweist, daß eine 1%ige w/v wäßrige Suspension der Stärke nach 1 Gefrier-Tau-Zyklus von Gefrieren über Nacht bei -70°C und Auftauen bei Raumtemperatur über mindestens zwei Stunden eine Extinktion von weniger als 0,5 Einheiten bei einer Wellenlänge von 700 nm aufweist.

17. Kartoffelpflanze nach Anspruch 8, die zu einer Stärke gemäß Anspruch 16 führt und in der eine 1%ige w/v wäßrige Suspension der Stärke nach 1 Gefrier-Tau-Zyklus eine Extinktion von weniger als 0,3 Einheiten bei einer Wellenlänge von 700 nm aufweist.

18. Kartoffelpflanze nach Anspruch 8, die zu einer Stärke gemäß Anspruch 9 führt und die bei Extraktion eine derartige Gefrier-Tau-Stabilität aufweist, daß eine 5%ige w/v wäßrige Paste der Stärke nach 4 Gefrier-Tau-Zyklen von Gefrieren über Nacht bei -70°C und Auftauen über 60 Minuten bei 22°C und anschließendem 10minütigem Zentrifugieren bei 8000 g bei 18°C eine Synärese von weniger als 40% aufweist.

19. Kartoffelpflanze nach Anspruch 8, die zu einer Stärke nach Anspruch 18 führt, die nach 4 Gefrier-Tau-Zyklen eine Synärese von weniger als 30% aufweist.

20. Kartoffelpflanze nach Anspruch 8, die zu einer Stärke nach Anspruch 18 führt, die nach 4 Gefrier-Tau-Zyklen eine Synärese von weniger als 20% aufweist.

21. Kartoffelpflanze nach Anspruch 8, die zu einer Stärke nach Anspruch 18 führt, die nach 4 Gefrier-Tau-Zyklen eine Synärese von weniger als 10% aufweist.

22. Kartoffelpflanze nach Anspruch 8, die zu einer Stärke gemäß Anspruch 9 führt und die bei Extraktion eine derartige Gefrier-Tau-Stabilität aufweist, daß eine 5%ige w/v wäßrige Paste der Stärke nach 3 Gefrier-Tau-Zyklen von Gefrieren über Nacht bei -70°C und Auftauen über 60 Minuten bei 22°C und anschließendem 10minütigem Zentrifugieren bei 8000 g bei 18°C eine Synärese von weniger als 30% aufweist.

23. Kartoffelpflanze nach Anspruch 8, die zu einer Stärke nach Anspruch 22 führt, die nach 3 Gefrier-Tau-Zyklen eine Synärese von weniger als 20% aufweist.

24. Kartoffelpflanze nach Anspruch 8, die zu einer Stärke nach Anspruch 22 führt, die nach 3 Gefrier-Tau-Zyklen eine Synärese von weniger als 10% aufweist.

25. Kartoffelpflanze nach Anspruch 8, die zu einer Stärke gemäß Anspruch 9 führt und die bei Extraktion eine derartige Gefrier-Tau-Stabilität aufweist, daß eine 5%ige w/v wäßrige Paste der Stärke nach 2 Gefrier-Tau-Zyklen von Gefrieren über Nacht bei -70°C und Auftauen über 60 Minuten bei 22°C und anschließendem 10minütigem Zentrifugieren bei 8000 g bei 18°C eine Synärese von weniger als 30% aufweist.

26. Kartoffelpflanze nach Anspruch 8, die zu einer Stärke nach Anspruch 25 führt, die nach 2 Gefrier-Tau-Zyklen eine Synärese von weniger als 20% aufweist.

27. Kartoffelpflanze nach Anspruch 8, die zu einer Stärke nach Anspruch 25 führt, die nach 2 Gefrier-Tau-Zyklen eine Synärese von weniger als 10% aufweist.

28. Kartoffelpflanze nach Anspruch 8, die zu einer Stärke gemäß Anspruch 9 führt und die bei Extraktion eine derartige Gefrier-Tau-Stabilität aufweist, daß eine 5%ige w/v wäßrige Paste der Stärke nach 4 Gefrier-Tau-Zyklen von Gefrieren über 1 Stunde bei -70°C und Auftauen über 10 Minuten bei 22°C und anschließendem 10minütigem Zentrifugieren bei 8000 g bei 18°C eine Synärese von weniger als 40% aufweist.

29. Kartoffelpflanze nach Anspruch 8, die zu einer Stärke nach Anspruch 28 führt, die nach 4 Gefrier-Tau-Zyklen eine Synärese von weniger als 30% aufweist.

30. Kartoffelpflanze nach Anspruch 8, die zu einer Stärke nach Anspruch 28 führt, die nach 4 Gefrier-Tau-Zyklen eine Synärese von weniger als 20% aufweist.

31. Kartoffelpflanze nach Anspruch 8, die zu einer Stärke gemäß Anspruch 9 führt und die bei Extraktion eine derartige Gefrier-Tau-Stabilität aufweist, daß eine 5%ige w/v wäßrige Paste der Stärke nach 3 Gefrier-Tau-Zyklen von Gefrieren über 1 Stunde bei -70°C und Auftauen über 10 Minuten bei 22°C und anschließendem 10minütigem Zentrifugieren bei 8000 g bei 18°C eine Synärese von weniger als 40% aufweist.

32. Kartoffelpflanze nach Anspruch 8, die zu einer Stärke nach Anspruch 31 führt, die nach 3 Gefrier-Tau-Zyklen eine Synärese von weniger als 30% aufweist.

33. Kartoffelpflanze nach Anspruch 8, die zu einer Stärke nach Anspruch 31 führt, die nach 3 Gefrier-Tau-Zyklen eine Synärese von weniger als 20% aufweist.

34. Kartoffelpflanze nach Anspruch 8, die zu einer Stärke nach Anspruch 31 führt, die nach 3 Gefrier-Tau-Zyklen eine Synärese von weniger als 10% aufweist.

35. Kartoffelpflanze nach Anspruch 8, die zu einer Stärke gemäß Anspruch 9 führt und die bei Extraktion eine derartige Gefrier-Tau-Stabilität aufweist, daß eine 5%ige w/v wäßrige Paste der Stärke nach 2 Gefrier-Tau-Zyklen von Gefrieren über 1 Stunde bei -70°C und Auftauen über 10 Minuten bei 22°C und anschließendem 10minütigem Zentrifugieren bei 8000 g bei 18°C eine Synärese von weniger als 30% aufweist.

36. Kartoffelpflanze nach Anspruch 8, die zu einer Stärke nach Anspruch 29 führt, die nach 2 Gefrier-Tau-Zyklen eine Synärese von weniger als 20% aufweist.

37. Kartoffelpflanze nach Anspruch 8, die zu einer Stärke nach Anspruch 35 führt, die nach 2 Gefrier-Tau-Zyklen eine Synärese von weniger als 10% aufweist.

38. Kartoffelpflanze nach Anspruch 8, die zu einer Stärke nach Anspruch 9 führt, die bei Extraktion ein Verhältnis von kurzkettigen Fraktion-1-Glukanen zu kurzkettigen Fraktion-II-Glukanen von mindestens 60% aufweist.

39. Kartoffelpflanze nach Anspruch 8, die zu einer Stärke nach Anspruch 38 führt, bei der das Verhältnis von Fraktion-I zu Fraktion-II mindestens 65% beträgt.

40. Kartoffelpflanze nach Anspruch 8, die zu einer Stärke nach Anspruch 38 führt, bei der das Verhältnis von Fraktion-I zu Fraktion-II mindestens 70% beträgt.

41. Kartoffelpflanze nach Anspruch 8, die zu einer Stärke nach Anspruch 9 führt, bei der die Viskositäts-Onset-Temperatur weniger als 65°C beträgt.

42. Kartoffelpflanze nach Anspruch 8, die zu einer Stärke nach Anspruch 9 führt, bei der die Viskositäts-Onset-Temperatur weniger als 55°C beträgt.

43. Kartoffelpflanze nach Anspruch 8, die zu einer Stärke nach Anspruch 9 führt, bei der die Viskositäts-Onset-Temperatur weniger als 51°C beträgt.

44. Kartoffelpflanze nach Anspruch 8, die zu einer Stärke nach Anspruch 9 führt, bei der bei der Analyse mittels Differential Scanning Calorimetry mit einem Perkin Elmer DSC7-Gerät eine Stärkeprobe von 10 mg in einer wäßrigen Mischung von weniger als 25% Stärke w/v eine Verkleisterungs-Onset-Temperatur von weniger als 67°C aufweist.

45. Kartoffelpflanze nach Anspruch 8, die zu einer Stärke nach Anspruch 44 führt, die eine Verkleisterungs-Onset-Temperatur von weniger als 66°C aufweist.

46. Kartoffelpflanze nach Anspruch 8, die zu einer Stärke nach Anspruch 44 führt, die eine Verkleisterungs-Onset-Temperatur von weniger als 51°C aufweist.

47. Kartoffelpflanze nach Anspruch 8, die zu einer Stärke nach Anspruch 44 führt, die eine Verkleisterungs-Onset-Temperatur von weniger als 50°C aufweist.

48. Kartoffelpflanze nach Anspruch 8, die zu einer Stärke nach einem der Ansprüche 8-47 führt, wobei die Stärkekörner frei von Sprüngen sind.

49. Verfahren zur Änderung des Stärkegehalts einer Kartoffelpflanze, das die folgenden Schritte umfaßt: Bereitstellen von Nukleinsäuresequenzen; sowie Einführen dieser Sequenzen in die Kartoffelpflanze, wobei die Sequenzen spezifisch die Expression der GBSSI sowie mindestens eines weiteren Enzyms, das an der Stärkesynthese in der Kartoffelpflanze beteiligt ist, hemmen.

50. Verfahren nach Anspruch 49, wobei die Sequenzen in eine Kartoffelpflanzenzelle eingeführt werden und die Pflanzenzelle zu einem Kartoffelpflänzchen und anschließend zu einer Kartoffelpflanze herangezogen wird.

51. Verfahren nach Anspruch 49 oder 50, wobei die eingeführten Sequenzen spezifisch die Expression der GBSSI sowie der SSII und/oder SSIII hemmen.

52. Verfahren nach einem der Ansprüche 49-51, wobei die eingeführten Nukleinsäuresequenzen operativ in antisense-Richtung bezüglich eines in der Kartoffelpflanze aktiven Promoters verknüpft sind, um so die Transkription der Sequenzen zu bewirken.

53. Verfahren zur Herstellung von Stärke, das die folgenden Schritte umfaßt: Verändern des Stärkegehalts einer Kartoffelpflanze nach dem Verfahren von einem der Ansprüche 49-51; sowie Extrahieren des veränderten Stärkegehalts aus der Kartoffelpflanze.

54. Verfahren nach Anspruch 53, das weiterhin den Schritt umfaßt, daß man die extrahierte Stärke mittels physikalischem und/oder enzymatischem und/oder chemischem Bearbeiten in vitro modifiziert.

55. Kartoffelknolle, die von einer Kartoffelpflanzennachkommenschaft nach Anspruch 6 oder 7 erzeugt wird und eine Kartoffelpflanzenzelle nach den Ansprüche 1-3 umfaßt.

56. Kartoffelknolle, die von einer Kartoffelpflanze nach einem der Ansprüche 9-48 erzeugt wird und eine Kartoffelpflanzenzelle nach den Ansprüche 1-3 umfaßt.

## Revendications

1. Cellule de plante de pomme de terre comprenant des séquences d'acides nucléiques introduites, lesdites séquences inhibant spécifiquement l'expression de l'amidon synthase I liée aux granules (GBSSI) et d'au moins une enzyme amidon synthase supplémentaire impliquée dans la synthèse d'amidon dans la cellule de plante de pomme de terre.

2. Cellule de plante de pomme de terre selon la revendication 1, dans laquelle ladite au moins une enzyme amidon synthase supplémentaire est spécifiquement inhibée par les séquences d'acides nucléiques introduites comprenant l'amidon synthase II et/ou l'amidon synthase III.

3. Cellule de plante de pomme de terre selon la revendication 1, comprenant des séquences d'acides nucléiques introduites, lesdites séquences inhibant spécifiquement l'expression de trois ou plus de trois enzymes impliquées dans la synthèse d'amidon dans la cellule.

4. Plante de pomme de terre comprenant des séquences d'acides nucléiques introduites, les séquences d'acides nucléiques introduites inhibant spécifiquement l'expression de la GBSSI et d'au moins une enzyme amidon synthase supplémentaire impliquée dans la synthèse d'amidon dans la plante de pomme de terre.

5. Plante de pomme de terre selon la revendication 4, dans laquelle les séquences d'acides nucléiques introduites inhibent spécifiquement la GBSSI et l'une parmi la SSII et la SSIII ou les deux.

6. Descendance d'une plante de pomme de terre comprenant des séquences d'acides nucléiques introduites, ladite descendance conservant les séquences introduites, les séquences d'acides nucléiques introduites inhibant spécifiquement l'expression de la GBSSI et d'au moins une enzyme amidon synthase supplémentaire impliquée dans la synthèse d'amidon dans la plante de pomme de terre.

7. Descendance d'une plante de pomme de terre selon la revendication 6, dans laquelle les séquences d'acides nucléiques introduites inhibent spécifiquement la GBSSI et l'une parmi la SSII et la SSIII ou les deux.

8. Plante de pomme de terre selon la revendication 5, produite à partir d'une cellule de plante de pomme de terre selon l'une quelconque des revendications 1 à 3.

9. Plante de pomme de terre selon la revendication 8, qui donne de l'amidon dont la teneur apparente en amylose est inférieure à 8 %, telle que déterminée par la méthode de Morrison et Laignelet (1983 Cereal Science 1, 9-20) et dont la température de début de viscosité est inférieure à 67 °C, telle que déterminée par l'analyse viscosimétrique d'une suspension aqueuse à 7,4 % (p/v) de l'amidon à l'aide d'un instrument Rapid Visco Amylograph de Newport Scientific Série 4 fonctionnant selon le protocole de chauffage et d'agitation standard 1.

10. Plante de pomme de terre selon la revendication 8, qui donne de l'amidon tel que défini dans la revendication 9 et qui, lorsqu'il est extrait, affiche une stabilité à la congélationdécongélation telle qu'une suspension aqueuse à 1 % p/v de l'amidon présente une absorbance à une longueur d'onde de 700 nm inférieure à 1,2 unité suite à 4 cycles de congélation/décongélation comprenant une congélation à -70 °C pendant une nuit et une décongélation à température ambiante pendant au moins deux heures.

11. Plante de pomme de terre selon la revendication 8, qui donne de l'amidon tel que défini dans la revendication 9 et dans laquelle une suspension aqueuse à 1 % p/v de l'amidon présente une absorbance à une longueur d'onde de 700 nm inférieure à 1,0 unité suite à 4 cycles de congélation/décongélation.

12. Plante de pomme de terre selon la revendication 8, qui donne de l'amidon tel que défini dans la revendication 9 qui, lorsqu'il est extrait, affiche une stabilité à la congélationdécongélation telle qu'une suspension aqueuse à 1 % p/v de l'amidon présente une absorbance à une longueur d'onde de 700 nm inférieure à 0,9 unité suite à 3 cycles de congélation/décongélation comprenant une congélation à -70 °C pendant une nuit et une décongélation à température ambiante pendant au moins deux heures.

13. Plante de pomme de terre selon la revendication 8, qui donne de l'amidon tel que défini dans la revendication 12, dans laquelle une suspension aqueuse à 1 % p/v de l'amidon présente une absorbance à une longueur d'onde de 700 nm inférieure à 0,7 unité suite à 3 cycles de congélation/décongélation.

14. Plante de pomme de terre selon la revendication 8, qui donne de l'amidon tel que défini dans la revendication 9 qui, lorsqu'il est extrait, affiche une - stabilité à la congélationdécongélation telle qu'une suspension aqueuse à 1 % p/v de l'amidon présente une absorbance à une longueur d'onde de 700 nm inférieure à 0,7 unité suite à 2 cycles de congélation/décongélation comprenant une congélation à -70 °C pendant une nuit et une décongélation à température ambiante pendant au moins deux heures.

15. Plante de pomme de terre selon la revendication 8, qui donne de l'amidon tel que défini dans la revendication 14, dans laquelle une suspension aqueuse à 1 % p/v de l'amidon présente une absorbance à une longueur d'onde de 700 nm inférieure à 0,5 unité suite à 2 cycles de congélation/décongélation.

16. Plante de pomme de terre selon la revendication 8, qui donne de l'amidon tel que défini dans la revendication 9 qui, lorsqu'il est extrait, affiche une stabilité à la congélationdécongélation telle qu'une suspension aqueuse à 1 % p/v de l'amidon présente une absorbance à une longueur d'onde de 700 nm inférieure à 0,5 unité suite à 1 cycle de congélation/décongélation comprenant une congélation à -70 °C pendant une nuit et une décongélation à température ambiante pendant au moins deux heures.

17. Plante de pomme de terre selon la revendication 8, qui donne de l'amidon tel que défini dans la revendication 16, dans laquelle une suspension aqueuse à 1 % p/v de l'amidon présente une absorbance à une longueur d'onde de 700 nm inférieure à 0,3 unité suite à 1 cycle de congélation/décongélation.

18. Plante de pomme de terre selon la revendication 8, qui donne de l'amidon tel que défini dans la revendication 9 qui, lorsqu'il est extrait, affiche une stabilité à la congélationdécongélation telle qu'une pâte aqueuse à 5 % p/v de l'amidon affiche moins de 40 % de synérèse suite à 4 cycles de congélation/décongélation comprenant une congélation à -70 °C pendant une nuit et une décongélation à 22 °C pendant 60 minutes, suivis d'une centrifugation à 8 000 g pendant 10 minutes à 18 °C.

19. Plante de pomme de terre selon la revendication 8, qui donne de l'amidon tel que défini dans la revendication 18 qui affiche moins de 30 % de synérèse suite à 4 cycles de congélation/décongélation.

20. Plante de pomme de terre selon la revendication 8, qui donne de l'amidon tel que défini dans la revendication 18 qui affiche moins de 20 % de synérèse suite à 4 cycles de congélation/décongélation.

21. Plante de pomme de terre selon la revendication 8, qui donne de l'amidon tel que défini dans la revendication 18 qui affiche moins de 10 % de synérèse suite à 4 cycles de congélation/décongélation.

22. Plante de pomme de terre selon la revendication 8, qui donne de l'amidon tel que défini dans la revendication 9 qui, lorsqu'il est extrait, affiche une stabilité à la congélationdécongélation telle qu'une pâte aqueuse à 5 % p/v de l'amidon affiche moins de 30 % de synérèse suite à 3 cycles de congélation/décongélation comprenant une congélation à -70 °C pendant une nuit et une décongélation à 22 °C pendant 60 minutes, suivis d'une centrifugation à 8 000 g pendant 10 minutes à 18 °C.

23. Plante de pomme de terre selon la revendication 8, qui donne de l'amidon tel que défini dans la revendication 22 qui affiche moins de 20 % de synérèse suite à 3 cycles de congélation/décongélation.

24. Plante de pomme de terre selon la revendication 8, qui donne de l'amidon tel que défini dans la revendication 22 qui affiche moins de 10 % de synérèse suite à 3 cycles de congélation/décongélation.

25. Plante de pomme de terre selon la revendication 8, qui donne de l'amidon tel que défini dans la revendication 9 qui, lorsqu'il est extrait, affiche une stabilité à la congélationdécongélation telle qu'une pâte aqueuse à 5 % p/v de l'amidon affiche moins de 30 % de synérèse suite à 2 cycles de congélation/décongélation comprenant une congélation à -70 °C pendant une nuit et une décongélation à 22 °C pendant 60 minutes, suivis d'une centrifugation à 8 000 g pendant 10 minutes à 18 °C.

26. Plante de pomme de terre selon la revendication 8, qui donne de l'amidon tel que défini dans la revendication 25 qui affiche moins de 20 % de synérèse suite à 2 cycles de congélation/décongélation.

27. Plante de pomme de terre selon la revendication 8, qui donne de l'amidon tel que défini dans la revendication 25 qui affiche moins de 10 % de synérèse suite à 2 cycles de congélation/décongélation.

28. Plante de pomme de terre selon la revendication 8, qui donne de l'amidon tel que défini dans la revendication 9 qui, lorsqu'il est extrait, affiche une stabilité à la congélationdécongélation telle qu'une pâte aqueuse à 5 % p/v de l'amidon affiche moins de 40 % de synérèse suite à 4 cycles de congélation/décongélation comprenant une congélation à -70 °C pendant 1 heure et une décongélation à 22 °C pendant 10 minutes, suivis d'une centrifugation à 8 000 g pendant 10 minutes à 18 °C.

29. Plante de pomme de terre selon la revendication 8, qui donne de l'amidon tel que défini dans la revendication 28 qui affiche moins de 30 % de synérèse suite à 4 cycles de congélation/décongélation.

30. Plante de pomme de terre selon la revendication 8, qui donne de l'amidon tel que défini dans la revendication 28 qui affiche moins de 20 % de synérèse suite à 4 cycles de congélation/décongélation.

31. Plante de pomme de terre selon la revendication 8, qui donne de l'amidon tel que défini dans la revendication 9 qui, lorsqu'il est extrait, affiche une stabilité à la congélationdécongélation telle qu'une pâte aqueuse à 5 % p/v de l'amidon affiche moins de 40 % de synérèse suite à 3 cycles de congélation/décongélation comprenant une congélation à -70 °C pendant 1 heure et une décongélation à 22 °C pendant 10 minutes, suivis d'une centrifugation à 8 000 g pendant 10 minutes à 18 °C.

32. Plante de pomme de terre selon la revendication 8, qui donne de l'amidon tel que défini dans la revendication 31 qui affiche moins de 30 % de synérèse suite à 3 cycles de congélation/décongélation.

33. Plante de pomme de terre selon la revendication 8, qui donne de l'amidon tel que défini dans la revendication 31 qui affiche moins de 20 % de synérèse suite à 3 cycles de congélation/décongélation.

34. Plante de pomme de terre selon la revendication 8, qui donne de l'amidon tel que défini dans la revendication 31 qui affiche moins de 10 % de synérèse suite à 3 cycles de congélation/décongélation.

35. Plante de pomme de terre selon la revendication 8, qui donne de l'amidon tel que défini dans la revendication 9 qui, lorsqu'il est extrait, affiche une stabilité à la congélationdécongélation telle qu'une pâte aqueuse à 5 % p/v de l'amidon affiche moins de 30 % de synérèse suite à 2 cycles de congélation/décongélation comprenant une congélation à -70 °C pendant 1 heure et une décongélation à 22 °C pendant 10 minutes, suivis d'une centrifugation à 8 000 g pendant 10 minutes à 18 °C.

36. Plante de pomme de terre selon la revendication 8, qui donne de l'amidon tel que défini dans la revendication 29 qui affiche moins de 20 % de synérèse suite à 2 cycles de congélation/décongélation.

37. Plante de pomme de terre selon la revendication 8, qui donne de l'amidon tel que défini dans la revendication 35 qui affiche moins de 10 % de synérèse suite à 2 cycles de congélation/décongélation.

38. Plante de pomme de terre selon la revendication 8, qui donne de l'amidon tel que défini dans la revendication 9 qui, lorsqu'il est extrait, présente une proportion de glucanes à chaînes courtes de la fraction I par rapport à la fraction II d'au moins 60 %.

39. Plante de pomme de terre selon la revendication 8, qui donne de l'amidon tel que défini dans la revendication 38 qui présente une proportion de la fraction I par rapport à la fraction II d'au moins 65 %.

40. Plante de pomme de terre selon la revendication 8, qui donne de l'amidon tel que défini dans la revendication 38 qui présente une proportion de la fraction I par rapport à la fraction II d'au moins 70 %.

41. Plante de pomme de terre selon la revendication 8, qui donne de l'amidon tel que défini dans la revendication 9 qui présente une température de début de viscosité inférieure à 65 °C.

42. Plante de pomme de terre selon la revendication 8, qui donne de l'amidon tel que défini dans la revendication 9 qui présente une température de début de viscosité inférieure à 55 °C.

43. Plante de pomme de terre selon la revendication 8, qui donne de l'amidon tel que défini dans la revendication 9 qui présente une température de début de viscosité inférieure à 51 °C.

44. Plante de pomme de terre selon la revendication 8, qui donne de l'amidon tel que défini dans la revendication 9, dans laquelle, lorsqu'il est analysé par calorimétrie différentielle à balayage à l'aide d'un instrument Perkin Elmer DSC7, un échantillon d'amidon de 10 mg dans un mélange aqueux à moins de 25 % p/v d'amidon affiche une température de début de gélatinisation inférieure à 67 °C.

45. Plante de pomme de terre selon la revendication 8, qui donne de l'amidon tel que défini dans la revendication 44 qui affiche une température de début de gélatinisation inférieure à 66 °C.

46. Plante de pomme de terre selon la revendication 8, qui donne de l'amidon tel que défini dans la revendication 44 qui affiche une température de début de gélatinisation inférieure à 51 °C.

47. Plante de pomme de terre selon la revendication 8, qui donne de l'amidon tel que défini dans la revendication 44 qui affiche une température de début de gélatinisation inférieure à 50 °C.

48. Plante de pomme de terre selon la revendication 8, qui donne de l'amidon tel que défini dans l'une quelconque des revendications 8 à 47, dans laquelle les granules d'amidon sont exempts de craquelures.

49. Procédé de modification de la teneur en amidon d'une plante de pomme de terre, le procédé comprenant les étapes consistant : à obtenir des séquences d'acides nucléiques ; et à introduire lesdites séquences dans la plante de pomme de terre, les séquences inhibant spécifiquement l'expression de la GBSSI et d'au moins une enzyme supplémentaire impliquée dans la synthèse d'amidon dans la plante de pomme de terre.

50. Procédé selon la revendication 49, selon lequel les séquences sont introduites dans une cellule de plante de pomme de terre et la cellule de plante se développe en une plantule de pomme de terre et par la suite en une plante de pomme de terre.

51. Procédé selon la revendication 49 ou 50, selon lequel les séquences introduites inhibent spécifiquement l'expression de la GBSSI et d'une parmi la SSII et la SSIII ou les deux.

52. Procédé selon l'une quelconque des revendications 49 à 51, selon lequel les séquences d'acides nucléiques introduites sont liées de façon opérationnelle en orientation antisens à un promoteur actif dans la plante de pomme de terre, de manière à provoquer la transcription des séquences.

53. Procédé de fabrication d'amidon, le procédé comprenant les étapes consistant : à modifier la teneur en amidon d'une plante de pomme de terre par le procédé selon l'une quelconque des revendications 49 à 51 ; et à extraire la teneur en amidon modifiée de la plante de pomme de terre.

54. Procédé selon la revendication 53, comprenant en outre l'étape consistant à transformer l'amidon extrait par traitement physique et/ou enzymatique et/ou chimique *in vitro.*

55. Tubercule de pomme de terre produit par une descendance de plante de pomme de terre selon la revendication 6 ou 7 et comprenant une cellule de plante de pomme de terre selon les revendications 1 à 3.

56. Tubercule de pomme de terre produit par une plante de pomme de terre selon l'une quelconque des revendications 9 à 48 et comprenant une cellule de plante de pomme de terre selon les revendications 1 à 3.
